# EUROPÄISCHE PATENTSCHRIFT

(11) **EP 0 366 004 B1**
(45) Veröffentlichungstag und Bekanntmachung des Hinweises auf die Patenterteilung: **30.03.1994**
(21) Anmeldenummer: 89119411.0
(22) Anmeldetag: 19.10.1989
(51) Int. Cl.: C07D 207/16, C07D 211/60, C07D 211/62, C07D 211/58, C07D 211/22, C07D 211/46, A61K 31/40, A61K 31/445

(54) **Cyclische Azaaliphaten mit Nitroxyfunktion, Verfahren zu ihrer Herstellung und diese Verbindungen enthaltende Arzneimittel**
Cyclic azaaliphatic compounds with a nitroxy group, process for their preparation and pharmaceutical compositions thereof
Composés azaaliphatiques cycliques avec un groupe nitroxyle, leur procédé de préparation et compositions pharmaceutiques les contenant

(30) Priorität: 22.10.1988 DE 3836084
(43) Veröffentlichungstag der Anmeldung: 02.05.1990
(73) Patentinhaber: BOEHRINGER MANNHEIM GMBH, 68298 Mannheim (DE)
(72) Erfinder: Simon, Herbert, Dr.rer.nat., D-6840 Lampertheim (DE); Michel, Helmut, D-6800 Mannheim 31 (DE); Schultz, Michael, Dr.rer.nat., D-6800 Mannheim 1 (DE); Bartsch, Wolfgang, Dr.med.vet., D-6806 Viernheim (DE)

(56) Entgegenhaltungen:
- EP-A- 0 101 093
- EP-A- 0 280 951
- US-A- 3 869 463
- CHEMICAL ABSTRACTS, Band 108, Nr. 21, 1988, Seite 688, Zusammenfassung Nr. 186566r, Columbus, Ohio, US;
- CHEMICAL ABSTRACTS, Band 103, Nr. 17, 1985, Seite 686, Zusammenfassung Nr. 141568y, Columbus, Ohio, US; F. LEVENDECKER et al.: "Factors controlling enantioselective recognition of chalcone by chiral cuprates. I: chiral auxiliaries derived from 1.-tartaric acid and 1.-proline",

## Beschreibung

Gegenstand der vorliegenden Erfindung sind cyclische Azaaliphaten der allgemeinen Formel I 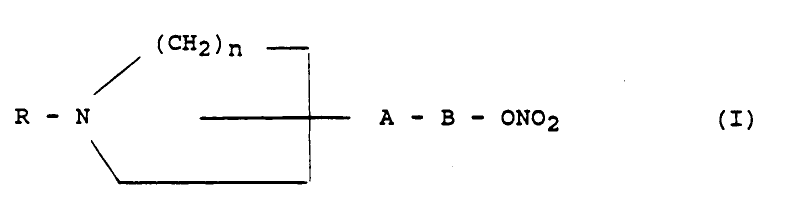
worin
- n: 1 oder 2 bedeutet,
- R: Wasserstoff, H-(C₁-C₈)-Alkylen, Hydroxy-(C₁-C₈)-Alkylen, R¹R²N-(C₁-C₈)-Alkylen, R¹R²N-(C₁-C₈)-Alkylen-CO-, R¹R²N-CO-NR³-(C₁-C₈)-Alkylen, oder R₁R₂N-CO- bedeutet, worin
- R¹: Wasserstoff oder eine geradkettige oder verzweigte, gesättigte oder ungesättigte, oder cyclische Alkyl-Gruppe von 1-6 C-Atomen bedeutet,
- R²: Wasserstoff oder eine geradkettige oder verzweigte, gesättigte oder ungesättigte, oder cyclische Alkyl-Gruppe von 1-6 C-Atomen bedeutet, oder R² gemeinsam mit einer - (CH₂)-Gruppe der benachbarten (C₁-C₈)-Alkyl-Gruppe und dem Stickstoff-Atom einen heteroaliphatischen Ring von 2-6 C-Atomen aufspannen kann,
- R³: Wasserstoff oder eine geradkettige oder verzweigte, gesättigte oder ungesättigte, oder cyclische Alkyl-Gruppe von 1-6 C-Atomen bedeutet,
- A: eine Bindung, eine -NR⁴-CO-Gruppe oder eine -CO-NR⁴-Gruppe bedeutet,
worin
- R⁴: Wasserstoff oder eine geradkettige oder verzweigte, gesättigte oder ungesättigte oder cyclische Alkyl-Gruppe von 1-6 C-Atomen bedeutet,
- B: eine Bindung oder eine geradkettige oder verzweigte Alkylenkette von 1-8 C-Atomen, wobei eine -CH₂-Gruppe durch eine Cycloalkylen-Gruppe mit 3-7 C-Atomen ersetzt sein kann, bedeutet,
mit der Maßgabe, daß R keine R₁R₂N-(C₁-C₈)-Alkylen-CO-Gruppe sein kann, in der R₁ und R₂ gleichzeitig Wasserstoff bedeuten, und der Maßgabe, daß R keine H-(C₁-C₈)-Alkylgruppe bedeutet, wenn A und B gleichzeitig eine Bindung darstellen und n die Zahl 2 bedeutet,
sowie deren optisch aktive Formen und physiologisch verträgliche Salze.

Die erfindungsgemäßen Verbindungen der allgemeinen Formel I besitzen wertvolle Eigenschaften. Sie bewirken eine Verminderung des Sauerstoffbedarfs des Herzens, eine Erhöhung des Blutflusses und eine Erniedrigung des Blutdrucks. Überraschenderweise wurde nun gefunden, daß die beanspruchten Verbindungen eine nitratartige Wirkung von besonders langer Wirkdauer aufweisen. Sie eignen sich daher zur Prophylase und/oder Behandlung von Herz- und Kreislauferkrankungen, wie z.B. Angina pectoris.

Ähnliche Verbindungen der allgemeinen Formel I, in der R eine Amino-(C₁-C₈)-alkylen-carbonyl-gruppe ist, sind bereits aus EP-A-0,280,951 bekannt. Sie sind dort als Verbindungen der allgemeinen Formel IV beschrieben, und stellen lediglich Zwischenprodukte zur Herstellung von Aryloxy-aminopropanol-Derivaten dar. Eine pharmakologische Wirkung dieser Verbindungen war bisher nicht bekannt.

Azaaliphatische Verbindungen mit n=2 (Piperidin-Derivate), in denen A und B gleichzeitig eine Bindung darstellen und R eine C₁-C₂₀-Alkylgruppe bedeutet, sind aus EP-A-101,093 (Ethyl Corp.) als Zusatzmittel für Dieselkraftstoffe bekannt, insbesondere die Verbindungen 1-Methyl-3-nitroxy-piperidin und 1-Methyl-4-nitroxy-piperidin. Ferner ist die Verbindung 1-Methyl-4-nitroxy-piperidin in US 4 405 334 (CA 100, P 9842 S) beschrieben.

Für die aus dem Stand der Technik bekannten Verbindungen ist bisher noch keine pharmakologische Wirkung beschrieben worden, so daß Gegenstand der vorliegenden Erfindung auch Arzneimittel sind, die sowohl diese bekannten Verbindungen enthalten, als auch die neuen Verbindungen der Formel I.

R kann Wasserstoff oder H-(C₁-C₈)-Alkylen, Hydroxy-(C₁-C₈)-Alkylen, R¹R²N-(C₁-C₈)-Alkylen, R¹R²N-(C₁-C₈)-Alkylen-CO-, R₁R₂N-CO oder R¹R²N-CO-NR³-(C₁-C₈)-Alkylen bedeuten. Für (C₁-C₈)-Alkylen kommt dabei eine geradkettige, verzweigte oder cyclische Alkylenkette von 1-8 C-Atomen in Frage, bevorzugt Methylen, Ethylen, Trimethylen, Tetramethylen, Pentamethylen, ferner Hexamethylen, Heptamethylen oder Octamethylen. (C₁-C₈)-Alkylen kann auch verzweigt sein und aus 2-8, bevorzugt 2-5 C-Atomen bestehen. Bevorzugt sind z.B. Methylmethylen, 1-Methylethylen, Dimethylmethylen, 1,1-Dimethylethylen, 1,1-Dimethyltrimethylen, 1-Methyltrimethylen; ferner kommen z.B. 2,2-Dimethylethylen, 1-Ethylmethylen, 1,1-Dimethyltetramethylen, 1,2-Dimethyltrimethylen, 2,2-Dimethyltrimethylen, 3,3-Dimethyl-trimethylen, 1,1-Dimethyl-pentamethylen, 1,1-Dimethyl-hexamethylen, oder 1-Methyltetramethylen in Frage.

Die (C₁-C₈)-Alkylen-Gruppe kann auch cyclisch sein und aus 3-8, bevorzugt 6 C-Atomen bestehen. Vorzugsweise kommen der 1,2-, 1,3- und 1,4-Cyclohexylen-Rest in Frage, wobei die Stellung der Substituenten jeweils cis oder trans sein kann. Ferner kann z.B. die Methylen-1,2-, -1,3- oder -1,4-cyclohexylen-Gruppe, die Methylen-1,2-, -1,3- oder -1,4-cyclohexylen-methylen-Gruppe, die Ethylen-1,2-, -1,3- oder -1,4-cyclohexylen-Gruppe eingesetzt werden, die Konfiguration der Cycloalkylen-Gruppe kann jeweils cis oder trans sein. Weitere (C₁-C₈)-Alkylenketten sind z.B. die 1,2-Cyclopropylen-Gruppe, die Methylen-1,2-cyclopropylen-Gruppe, die Tetramethylen-1,2-cyclopropylen-Gruppe, die Methylen-1,2-cyclopropylen-methylen-Gruppe, die Methylen-1,2-cyclopropylen-ethylen-Gruppe, die 1,2- oder 1,3-Cyclobutylen-Gruppe, die Methylen-1,2- oder -1,3-cyclobutylen-Gruppe, die Tetramethylen-1,2- oder -1,3-cyclobutylen-Gruppe, die Methylen-1,2- oder -1,3-cyclobutylen-methylen-Gruppe, die Methylen-1,2- oder -1,3-cyclobutylen-ethylen-Gruppe, die 1,2- oder 1,3-Cyclopentylen-Gruppe, die Methylen-1,2- oder -1,3-cyclopentylen-Gruppe, die Methylen-1,2- oder -1,3-cyclopentylen-methylen-Gruppe, die Methylen-1,2- oder -1,3-cyclopentylen-ethylen-Gruppe, die 1,2-, 1,3- oder 1,4-Cycloheptylen-Gruppe, die Methylen-1,2-, -1,3- oder -1,4-cycloheptylen-Gruppe, die Konfiguration der Cycloalkylen-Gruppe kann jeweils cis oder trans sein.

R¹ kann Wasserstoff oder eine geradkettige, verzweigte oder cyclische Alkylkette von 1-6 C-Atomen, bevorzugt Methyl, Ethyl, n-Propyl, Isopropyl, n-Butyl, n-Pentyl, Cyclopropyl, Cyclopentyl und Cyclohexyl bedeuten, ferner kommen z.B. Isobutyl, tert.-Butyl, 2-Pentyl, 2-Hexyl und Cyclobutyl in Frage.

R² kann Wasserstoff oder eine geradkettige, verzweigte oder cyclische Alkylkette von 1-6 C-Atomen, bevorzugt Methyl, Ethyl, n-Propyl, Isopropyl, n-Butyl, n-Pentyl, n-Hexyl, Cyclopentyl oder Cyclohexyl bedeuten, ferner kommen z.B. 2-Butyl, Isobutyl, 2-Pentyl, 2-Hexyl oder Cyclopropyl in Frage. Darüberhinaus kann R² gemeinsam mit einer -(CH₂)-Gruppe der (C₁-C₈)-Alkylen-Gruppe und dem benachbarten Stickstoff-Atom einen heteroaliphatischen Ring von 2-6 C-Atomen aufspannen. Als Beispiele sind der Aziridin-Ring, der Azetidin-Ring, der Pyrrolidin-Ring, der Piperidin-Ring und der Perhydroazepin-Ring zu nennen.

R³ kann Wasserstoff oder eine geradkettige, verzweigte oder cyclische Alkylkette von 1-6 C-Atomen, bevorzugt Methyl, Ethyl, n-Propyl, Isopropyl, n-Butyl, n-Pentyl, Cyclopropyl, Cyclopentyl und Cyclohexyl bedeuten, ferner kommen z.B. Isobutyl, 2-Butyl, 2-Pentyl oder 2-Hexyl in Frage.

Die Gruppe A-B-ONO₂ kann für den Fall, daß n = 1 bedeutet, in 2- oder 3-Stellung des Pyrrolidinrings stehen, bevorzugt jedoch in 3-Stellung, und für den Fall, daß n = 2 bedeutet, in 2-, 3- oder 4-Stellung des Piperidinrings, wobei dei 4-Stellung bevorzugt ist.

R⁴ kann Wasserstoff oder eine geradkettige, verzweigte oder cyclische Alkylkette von 1-6 C-Atomen, z.B. Methyl, Ethyl, n-Propyl, Isopropyl, n-Butyl, n-Pentyl, Cyclopropyl, Cyclopentyl und Cyclohexyl bedeuten.

Die Gruppe B kann eine Bindung, eine geradkettige oder verzweigte Alkylenkette von 1-8 C-Atomen, vorzugsweise 2-6 C-Atomen, bedeuten, vorzugsweise Ethylen, 1-Methyl-ethylen, 2-Methyl-ethylen, Trimethylen, 1-Methyl-trimethylen, 3-Methyl-trimethylen, Tetramethylen, Pentamethylen, 1-Methyl-tetramethylen, 1-Ethyl-trimethylen, Hexamethylen, 1-Methyl-pentamethylen, 1-Ethyl-tetramethylen, 1-n-Propyl-tri-methylen, 1,1-Dimethyl-ethylen, ferner z.B. Dimethyl-methylen, 1,1-Dimethyl-tri-methylen, 2,2-Dimethyl-trimethylen, 2,2-Dimethyl-tetramethylen oder 1,1-Dimethyl-hexa-methylen.

Für den Fall, daß eine -(CH₂)-Gruppe der Kette B durch eine Cycloalkylen-Gruppe ersetzt ist, kommen bevorzugt, die 1,2-, 1,3- oder 1,4-Cyclohexylen-Gruppe in Frage, wobei die Konfiguration der Cyclohexylen-Gruppe jeweils cis oder trans sein kann.

Bevorzugt im Sinne der vorliegenden Erfindung sind solche Verbindungen, in denen
- R: ein Wasserstoffatom; eine H-(C₁-C₃)-Alkylengruppe, beispielsweise Methyl, Ethyl, n-Propyl oder iso-Propyl; eine HO-(C₁-C₃)-Alkylengruppe, beispielsweise 2-Hydroxypropyl; eine R¹R²N-(C₁-C₃)-Alkylengruppe, beispielsweise Aminoethylen oder Aminopropylen; eine R¹R²N-(C₁-C₅)-Alkylencarbonyl-Gruppe, beispielsweise Aminomethylcarbonyl, Dimethylamino-methylcarbonyl, (2-Amino-1-methyl)-ethylcarbonyl, 2-Amino-ethylcarbonyl, 2-(N,N-Dimethylamino)-ethyl-carbonyl, (3-Amino-3-methyl)-butylcarbonyl, 1-Amino-ethyl-carbonyl oder (2-Amino-2-methyl)-propylcarbonyl; eine R¹R²N-CO-NR³-(C₁-C₃)-Alkylengruppe, beispielsweise Aminocarbonylaminoethyl; oder eine R¹R²N-CO-Gruppe, beispielsweise iso-Propylaminocarbonyl oder Aminocarbonyl bedeutet,
- R₁: Wasserstoff oder eine geradkettige oder verzweigte C₁-C₃-Alkylgruppe, bespielsweise Methyl, Ethyl oder iso-Propyl, darstellt,
- R₂: Wasserstoff oder eine geradkettige C₁-C₃-Alkylgruppe, beispielsweise Methyl oder Ethyl bedeutet oder R₂ gemeinsam mit einer CH₂-Gruppe einer benachbarten Alkylengruppe und dem Stickstoffatom einen Piperidinring bildet,
- R₃: ein Wasserstoffatom darstellt,
- A: eine Bindung, oder die Gruppe -NH-CO- oder -CO-NH- bedeutet,
- B: eine Bindung, eine geradkettige oder verzweigte C₁-C₅-Alkylengruppe, wie beispielsweise Methylen, Ethylen, Trimethylen oder 1,1-Dimethyl-ethylen; oder eine 1,4-Cyclohexylengruppe darstellt.

Die erfindungsgemäßen Verbindungen werden üblicherweise in Mengen von 20-500 mg pro Tag, bezogen auf 75 kg Körpergewicht, appliziert. Bevorzugt ist es, 1-2 mal pro Tag 1-2 Tabletten mit einem Wirkstoffgehalt von 10-300 mg zu verabreichen. Die Tabletten können auch retardiert sein, wodurch nur noch 1 mal pro Tag 1-2 Tabletten mit 20-600 mg Wirkstoff gegeben werden muß. Der Wirkstoff kann auch durch Injektion 1-8 mal pro Tag bzw. durch Dauerinfusion gegeben werden, wobei Mengen von 5-200 mg/Tag normalerweise ausreichen.

Die erfindungsgemäßen Verbindungen der allgemeinen Formel I können in an sich bekannter Weise dadurch hergestellt werden, daß man
1) eine Verbindung der allgemeinen Formel II 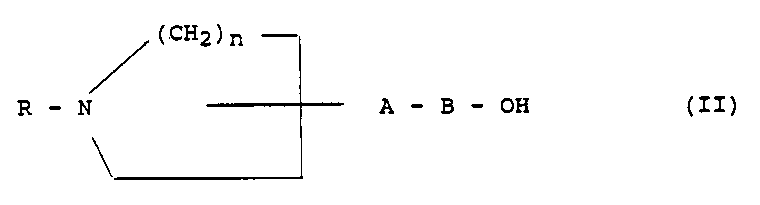 in der R, n, A und B die oben angegebenen Bedeutungen haben, einer Nitratester-Bildungsreaktion unterwirft, oder,
2.1) für den Fall, daß A eine -NR₄-CO-Gruppe darstellt, eine Verbindung der allgemeinen Formel III 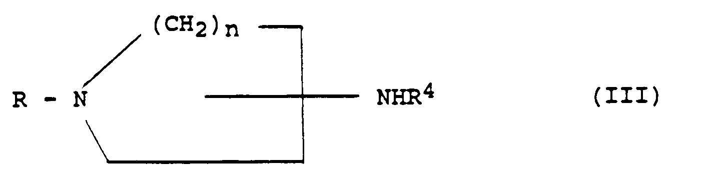 mit einer Verbindung der allgemeinen Formel IV

   Z - CO - B - ONO₂ (IV)

   wobei R, n, R⁴ und B die oben angegebenen Bedeutungen haben und Z eine nucleofuge Gruppe bedeutet, umsetzt, beziehungsweise
2.2) für den Fall, daß A eine -CO-NR⁴-Gruppe bedeutet, eine Verbindung der allgemeinen Formel V 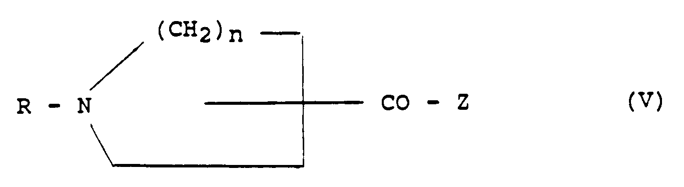 mit einer Verbindung der allgemeinen Formel VI

   HNR⁴ - B - ONO₂ (VI)

   wobei R, n, R⁴, B und Z die oben angebenen Bedeutungen haben, umsetzt, oder
2.3) im Fall, daß R nicht Wasserstoff bedeutet, eine Verbindung der allgemeinen Formel VII 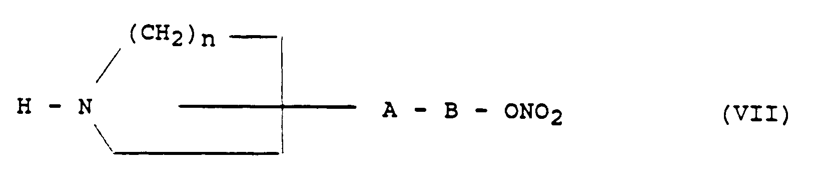 mit einer Verbindung der allgemeinen Formel VIII

   R - Z (VIII)

   wobei n, A, B, R und Z die oben an gegebenen Bedeutungen haben, umsetzt, oder
2.4) für den Fall, daß R die Gruppe R₁R₂N-CO- bedeutet, eine Verbindung der allgemeinen Formel VII mit Isocyanaten R₂-N=C=O oder Halogenameisensäure-Derivaten R₂-NH-CO-Hal umsetzt, wobei R₂, A und B die oben angegebene Bedeutung haben und Hal, Chlor oder Brom bedeutet, oder
2.5) für den Fall, daß R die Gruppe HR²N-CO-NR³-(C₁-C₈)-Alkylen- bedeutet, eine Verbindung der allgemeinen Formel IX 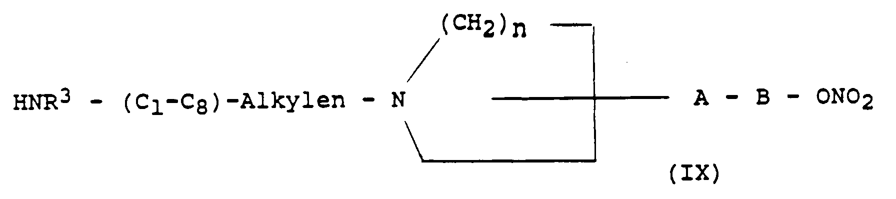 mit Isocyanaten R²-N=C=O oder Halogenameisensäure-Derivaten R²-NH-CO-Hal umsetzt, wobei R², R³, A, (C₁-C₈)-Alkylen und B die oben angegebenen Bedeutungen haben und Hal Chlor oder Brom bedeutet.

Die Verbindungen der allgemeinen Formel II, in der R nicht die Amino-(C₁-C₈)-Alkylen-carbonyl-Gruppe bedeutet, sind neu. Für den Fall, daß R eine Amino-(C₁-C₈)-Alkylen-carbonylgruppe darstellt, so sind diese Verbindungen aus EP-A-280,951 bereits in Form der dort in der allgemeinen Formel XIII beschriebenen Verbindungen bekannt.

Gegenstand der vorliegenden Erfindung sind deshalb auch die als Zwischenprodukte verwendbaren Verbindungen der allgemeinen Formel II 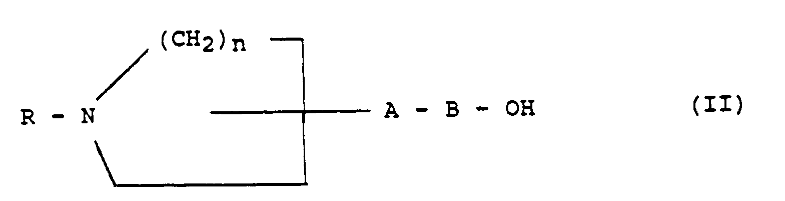
in der
- n: 1 oder 2 bedeutet,
- R: Wasserstoff, H-(C₁-C₈)-Alkylen, Hydroxy-(C₁-C₈)-Alkylen, R¹R²N-(C₁-C₈)-Alkylen, R¹R²N-(C₁-C₈)-Alkylen-CO-, R¹R²N-CO-NR³-(C₁-C₈)-Alkylen, oder R₁R₂N-CO- bedeutet, worin
- R¹: Wasserstoff oder eine geradkettige oder verzweigte, gesättigte oder ungesättigte, oder cyclische Alkyl-Gruppe von 1-6 C-Atomen bedeutet,
- R²: Wasserstoff oder eine geradkettige oder verzweigte, gesättigte oder ungesättigte, oder cyclische Alkyl-Gruppe von 1-6 C-Atomen bedeutet, oder R² gemeinsam mit einer -(CH₂)-Gruppe der benachbarten (C₁-C₈)-Alkyl-Gruppe und dem Stickstoff-Atom einen heteroaliphatischen Ring von 2-6 C-Atomen aufspannen kann,
- R³: Wasserstoff oder eine geradkettige oder verzweigte, gesättigte oder ungesättigte, oder cyclische Alkyl-Gruppe von 1-6 C-Atomen bedeutet,
- A: eine Bindung, eine -NR⁴-CO-Gruppe oder eine -CO-NR⁴-Gruppe bedeutet,
worin
- R⁴: Wasserstoff oder eine geradkettige oder verzweigte, gesättigte oder ungesättigte oder cyclische Alkyl-Gruppe von 1-6 C-Atomen bedeutet,
- B: eine Bindung oder eine geradkettige oder verzweigte Alkylenkette von 1-8 C-Atomen, wobei eine -CH₂-Gruppe durch eine Cycloalkylen-Gruppe mit 3-7 C-Atomen ersetzt sein kann, bedeutet,
mit der Maßgabe, daß R keine R₁R₂N-(C₁-C₈)-Alkylen-CO Gruppe sein kann, in der R₁ und R₂ gleichzeitig Wasserstoff bedeuten, der Maßgabe, daß R kein Wasserstoffatom oder eine H-(C₁-C₈)-Alkylengruppe bedeutet, wenn A eine Bindung darstellt und B eine Bindung oder eine Alkylenkette mit bis zu 2 C-Atomen bedeutet, und der Maßgabe, daß R keine CH₃- oder (CH₃)₃C-CH₂-Gruppe ist, wenn A eine Bindung, B eine Alkylenkette mit bis zu 2 C-Atomen und n gleich 1 bedeutet,
sowie deren optisch aktiven Formen.

Verbindungen der allgemeinen Formel II, in der R eine R₁R₂N-(C₁- C₈)-Alkylen-CO-Gruppe bedeutet sind aus EP-A-280,951 bekannt. Verbindungen, in denen R ein Wasserstoffatom oder eine H-(C₁-C₈)-Alkylengruppe, A eine Bindung und B eine Bindung oder eine Alkylengruppe mit bis zu 2 C-Atomen bedeutet, sind beschrieben in Chem.Abstr. 97 (13), 1982, 109 228j; Chem.Abstr. 103 (17), 1985, 141 568y; Chem.Abstr. 104 (11), 1986, 88 398k.

Verbindungen der allgemeinen Formel II können hergestellt werden, indem man in an sich bekannter Weise,
1) für den Fall, daß A eine -NR⁴-CO-Gruppe darstellt,
1.1) eine Verbindung der allgemeinen Formel III mit einer Verbindung der allgemeinen Formel X

   Z - CO - B - OH (X)

   wobei R, n, R⁴, B, und Z die oben angegebenen Bedeutungen haben, umsetzt, oder
1.2) eine Verbindung der allgemeinen Formel III mit einer Verbindung der allgemeinen Formel XI

   Z - CO - B - O - W (XI)

   wobei R, n, R⁴, B und Z die oben angegebenen Bedeutungen haben und W eine Schutzgruppe bedeutet, umsetzt und anschließend die Schutzgruppe W abspaltet, oder
1.3) eine Verbindung der allgemeinen Formel III mit einem Lacton der allgemeinen Formel XII 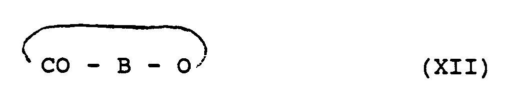 wobei R, n, R⁴ und B die oben angegebenen Bedeutungen haben, umsetzt, oder
1.4) eine Verbindung der allgemeinen Formel XIII 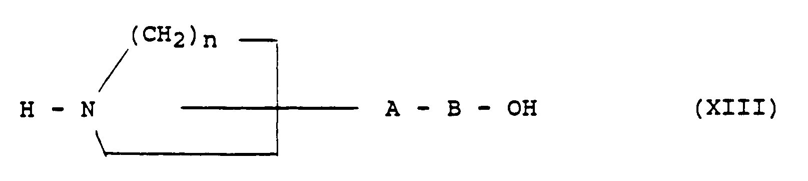 mit einer Verbindung der allgemeinen Formel VIII, wobei n, A, B, R und Z die oben angegebenen Bedeutungen haben, umsetzt, oder
1.5) für den Fall, daß R die Gruppe R¹R²N- enthält, eine Verbindung der allgemeinen Formel XIII mit einer Verbindung der allgemeinen Formel XIV

   V - R˝ - Z (XIV)

   wobei n, A, B und Z die oben angegebenen Bedeutungen haben,
   R˝ die Gruppen (C₁-C₈)-Alkylen, (C₁-C₈)-Alkylen-CO- oder (C₁-C₈)-Alkylen-CO-NR³-(C₁-C₈)-Alkylen bedeutet und V eine Schutzgruppe bedeutet, die in die Gruppe R¹R²N- überführt werden kann, umsetzt, oder
2) für den Fall, daß A eine -CO-NR₄-Gruppe darstellt,
2.1) eine Verbindung der allgemeinen Formel V mit einer Verbindung der allgemeinen Formel XV

   HNR⁴ - B - OH (XV)

   wobei R, n, Z, R⁴ und B die oben angegebenen Bedeutungen haben, umsetzt, oder
2.2) eine Verbindung der allgemeinen Formel V mit einer Verbindung der allgemeinen Formel XVI

   HNR⁴ - B - O - W (XVI)

   wobei R, n, Z, R⁴, B und W die oben genannten Bedeutungen haben, umsetzt, und anschließend die Schutzgruppe W abspaltet.

Die Nitratester-Bildungsreaktion der Verbindungen der allgemeinen Formeln II, X, XIII oder XV kann durchgeführt werden, indem die Verbindungen der allgemeinen Formel II, X, XIII oder XV mit einem nitratesterbildenden Reagenz, wie rauchender Salpetersäure, einer Mischung aus rauchender Salpetersäure und Acetanhydrid oder einer Mischung aus rauchender Salpetersäure und konz. Schwefelsäure bei niedrigen Temperaturen in Gegenwart oder Abwesenheit eines inerten Lösungsmittels umsetzt. Die Reaktionstemperaturen liegen zwischen Raumtemperatur und -60° C, bevorzugt zwischen -10° C und -30° C. Das Molverhältnis der Reaktionspartner liegt zwischen 1 und 10.

Alternativ kann die Nitratester-Bildungsreaktion durchgeführt werden, indem man in einer Verbindung der allgemeinen Formeln II, X, XIII oder XV selektiv die aliphatische Hydroxyl-Gruppe durch eine Halogen-Gruppe oder Alkyl- oder Arylsulfonsäureester-Gruppe ersetzt und anschließend das Reaktionsprodukt mit z.B. Silbernitrat oder Tetrabutylammoniumnitrat in Gegenwart oder Abwesenheit eines Lösungsmittels bei Temperaturen zwischen Raumtemperatur und 100° C umsetzt. Das Molverhaltnis der Umsetzungsreaktion zwischen der Halogenverbindung und Silbernitrat kann zwischen 1 und 10 liegen.

Als nucleofuge Gruppe Z kommen Alkoholate, Tosylate, Mesylate Halogenide oder Alkylcarboxylate in Frage. Die entsprechenden aktivierten Carbonsäuren IV, V, VIII, X, XI oder XIV liegen daher in Form von Estern, Carbonsäurehalogeniden oder - anhydriden vor.

Die Aktivierung der Carbonsäuren kann aber auch durch aktivierende Reagenzien wie N,N′-Carbonyldiimidazol, N,N′-Dicyclohexylcarbodiimid, 1-Ethyl-3-(3-dimethylaminopropyl)carbodiimid, 1-Alkyl-2-halogen-pyridiniumsalzen o.ä. Im Fall, daß es sich bei den Verbindungen der allgemeinen Formeln VIII oder XIV nicht um Carbon-säure-Derivate handelt, können diese als Halogenide, Tosylate oder Mesylate vorliegen. Die molaren Verhältnisse zwischen den Reaktionspartnern können zwischen 1 und 100 gewählt werden.

Als Schutzgruppe W wird vorwiegend die Acetyl-Gruppe verwendet. Daneben findet aber auch die Tetrahydropyranyl-, die Benzoyl- oder die Benzyl-Gruppe Verwendung. Die Abspaltung erfolgt gewöhnlich in saurer oder basischer, wäßriger oder alkoholischer Lösung.

Als Schutzgruppe V kommen z.B. die -NO₂-Gruppe, die Azido-Gruppe, die Benzyloxycarbonyl- oder eine durch 1 oder 2 Benzylreste geschützte Amin-Gruppe in Frage. Außerdem kann V eine nucleofuge Gruppe wie z.B. Halogenide, Mesylat oder Tosylat bedeuten, die mit Benzylgruppen-geschützten primären und sekundären Aminen umgesetzt werden oder den Aminen R¹R²NH umgesetzt werden. Zur Reduktion der -NO₂-Gruppe oder der Azido-Gruppe können zahlreiche Verfahren, z.B. mit Zink in Salzsäure, mit Eisen in Salzsäure, mit Lithiumaluminiumhydrid in Ethern, mit Natriumborhydrid in Alkoholen, mit Ammoniumformiat in Gegenwart von Edelmetallkatalysatoren wie Pd oder Pt, mit anorganischen Sulfiden wie NaHS, (NH₄)₂S oder Na₂S₂O₄ in wäßriger und/oder alkoholischer Lösung, oder hydrogenolytisch mit Katalysatoren wie PtO₂, Pd oder Raney-Nickel, vorzugsweise in einem alkoholischen Lösungsmittel wie Methanol oder Ethanol, bei Drucken zwischen 1 und 200 bar, angewendet werden. Die Temperaturen können zwischen -20° C und 200 °C, vorzugsweise zwischen Raumtemperatur und 100° C liegen. Die Umsetzung von Aminen mit Halogeniden, Mesylaten oder Tosylaten erfolgt in Gegenwart oder Abwesenheit eines Lösungsmitels zwischen 0° C und 150° C, bevorzugt zwischen 20° C und 80° C. Als Lösungsmittel können z.B. Methanol, Ethanol, Propanol, i-Propanol, Ether wie Diethylether, Diisopropylether, tert.-Butylmethylether oder Tetrahydrofuran oder aromatische Lösungsmittel wie Toluol oder Xylol verwendet werden. Das molare Verhältnis der Reaktionspartnerist nicht kritisch. Es können Verhältnisse zwischen 1 und 100 gewählt werden. Gegebenenfalls können die Reaktionen unter erhöhtem Druck durchgeführt werden. Die Abspaltung von Benzyl-Schutzgruppen erfolgt hydrogenolytisch in Gegenwart eines organischen Lösungsmittels und/oder Wasser sowie Palladium auf Kohle als Katalysator. Die Temperaturen können zwischen Raumtemperatur und 250° C, vorzugsweise bei Raumtemperatur und 60° C liegen, der Wasserstoffdruck kann zwischen 1 und 300 bar, vorzugsweise 1 bis 5 bar, betragen.

Die erfindungsgemäßen Verbindungen der allgemeinen Formeln I und II können asymmetrische Kohlenstoff-Atome besitzen. Gegenstand der Erfindung sind daher auch sämtliche möglichen Racemate und Diastereomerengemische sowie sämtliche optisch aktiven Formen der erfindungsgemäßen Verbindungen der allgemeinen Formel I und II.

Zur Überführung der Verbindungen der allgemeinen Formel I in ihre pharmakologisch unbedenklichen Salze setzt man diese, vorzugsweise in einem organischen Lösungsmittel, mit der äquivalenten Menge einer anorganischen oder organischen Säure, z.B. Salzsäure, Bromwasserstoffsäure, Salpetersäure, Phosphorsäure, Schwefelsäure, Ameisensäure, Essigsäure, Propionsäure, Oxalsäure, Fumarsäure, Maleinsäure, Bernsteinsäure, Adipinsäure, Benzoesäure, Salicylsäure, o-Acetoxybenzoesäure, Zimtsäure, Naphthoesäure, Mandelsäure, Citronensäure, Äpfelsäure, Weinsäure, Asparaginsäure, Glutaminsäure, Methansulfonsäure, p-Toluolsulfonsäure oder Cyclohexylsulfaminsäure um.

Die erfindungsgemäßen neuen Substanzen der allgemeinen Formel I und ihre Salze können in flüssiger und fester Form enteral oder parenteral appliziert werden. Als Injektionsmediumkommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- oder Citratpuffer, Ethanol, Komplexbildner (wie Ethylendiamintetraessigsäure oder deren nicht toxische Salze), hochmolekulare Polymere (wie flüssiges Polyethylenoxid) zur Viskositätsregulierung. Feste Trägerstoffe sind z.B. Stärke, Lactose, Mannit, Methylcellulose, Talkum hochdispers Kieselgelsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie z.B. Polyethylenglykole). Für orale Applikation geeignete Zubereitungen können gewünschtenfalls Geschmacks- und Süßstoffe enthalten.

Neben den nachfolgend aufgeführten Beispielen sind insbesondere die folgenden Verbindungen, sowie die entsprechenden Hydroxy-Verbindungen, die nicht nitratiert sind (Zwischenprodukt) im Sinne der Anmeldung bevorzugt:
N-(3-Nitroxy-propyl)-pyrrolidin-2-carbonsäureamid
1-Methyl-N-(2-methyl-1-nitroxy-prop-2-yl)-piperidin-2-carbonsäureamid
1-Methyl-N-(3-nitroxy-propyl)-piperidin-2-carbonsäureamid
1-Methyl-N-(2-methyl-1-nitroxy-prop-2-yl)-piperidin-3-carbonsäureamid
1-Methyl-N-(3-nitroxy-propyl)-piperidin-3-carbonsäureamid
1-Methyl-N-(4-nitroxy-2-methyl-but-2-yl)-piperidin-3-carbonsäureamid
1-Methyl-N-(1-nitroxy-2-methyl-prop-2-yl)-piperidin-4-carbonsäureamid
1-Methyl-N-(1-nitroxy-3-methyl-but-3-yl)-piperidin-4-carbonsäureamid
2-Nitroxy-N-(piperidin-4-yl)-propionsäureamid
2-Methyl-2-nitroxy-N-(piperidin-4-yl)-propionsäureamid
2,2-Dimethyl-3-nitroxy-N-(piperidin-4-yl)-propionsäureamid
2-Nitroxy-N-(1-methyl-piperidin-4-yl)-propionsäureamid
2-Methyl-2-nitroxy-N-(1-methyl-piperidin-4-yl)-propionsäureamid
trans-N-(2-Nitroxy-cyclohexyl)-pyrrolidin-2-carbonsäureamid
trans-N-(4-Nitroxy-cyclohexyl)-pyrrolidin-2-carbonsäureamid
trans-1-Methyl-N-(4-nitroxy-cyclohexyl)-piperidin-2-carbonsäureamid
trans-1-Methyl-N-(4-nitroxy-cyclohexyl)-piperidin-3-carbonsäureamid
trans-1-Methyl-N-(2-nitroxy-cyclohexyl)-piperidin-4-carbonsäureamid
trans-1-Methyl-N-(4-nitroxy-cyclohexyl)-piperidin-4-carbonsäureamid
cis-1-Methyl-N-(4-nitroxy-cyclohexyl)-piperidin-4-carbonsäureamid
trans-1-Methyl-N-(4-nitroxy-cyclohexylmethyl)-piperidin-4-carbonsäureamid
cis-1-Methyl-N-(4-nitroxy-cyclohexylmethyl)-piperidin-4-carbonsäureamid
trans-1-Methyl-N-(4-nitroxymethyl-cyclohexyl)-piperidin-4-carbonsäureamid
cis-1-Methyl-N-(4-nitroxymethyl-cyclohexyl)-piperidin-4-carbonsäureamid
trans-4-Nitroxy-N-(piperidin-4-yl)-cyclohexancarbonsäureamid
cis-4-Nitroxy-N-(piperidin-4-yl)-cyclohexancarbonsäureamid
cis-2-(4-Nitroxy-cyclohexyl)-N-(piperidin-4-yl)-essigsäureamid
cis-4-Nitroxy-N-(1-methyl-piperidin-4-yl)-cyclohexancarbonsäureamid
cis-2-(4-Nitroxy-cyclohexyl)-N-(1-methyl-piperidin-4-yl)-essigsäureamid
2-Amino-essigsäure-(4-nitroxymethyl)-piperidid
2-Amino-essigsäure-[4-(2-nitroxyethyl)]-piperidid
2-Amino-essigsäure-[4-(1-nitroxyethyl)]-piperidid
2-Dimethylamino-essigsäure-(4-nitroxymethyl)-piperidid
2-Dimethylamino-essigsäure-[4-(2-nitroxyethyl)]-piperidid
2-Dimethylamino-essigsäure-[4-(1-nitroxyethyl)]-piperidid
L-2-Amino-propionsäure-(4-nitroxy)-piperidid
L-2-Amino-propionsäure-(4-nitroxymethyl)-piperidid
L-2-Amino-propionsäure-[4-(2-nitroxyethyl)]-piperidid
3-Amino-3-methyl-buttersäure-(4-nitroxy)-piperidid
3-Dimethylamino-propionsäure-(4-nitroxymethyl)-piperidid
3-Dimethylamino-propionsäure-[4-(2-nitroxy-ethyl)]-piperidid
3-Amino-3-methyl-buttersäure-(4-nitroxymethyl)-piperidid
3-Amino-3-methyl-buttersäure-[4-(2-nitroxyethyl)]-piperidid
1-Methyl-piperidin-2-carbonsäure-(4-nitroxymethyl)-piperidid
1-Methyl-piperidin-3-carbonsäure-(4-nitroxymethyl)-piperidid
Piperidin-4-carbonsäure-(4-nitroxymethyl)-piperidid
Piperidin-4-carbonsäure-[4-(2-nitroxyethyl)]-piperidid
1-Methyl-piperidin-4-carbonsäure-(4-nitroxymethyl)-piperidid
1-Methyl-piperidin-4-carbonsäure-[4-(2-nitroxyethyl)]-piperidid
4-Nitroxy-1-(2-propyl)-piperidin

### Beispiel 1

### 1-Methyl-N-(2-nitroxy-ethyl)-piperidin-4-carbonsäureamid fumarat

10.2 g 1-Methyl-N-(2-hydroxy-ethyl)-piperidin-4-carbonsäureamid werden unter Rühren bei -10 bis -5°C in 23 ml 100proz. Salpetersäure eingetragen. Man läßt die Reaktionslösung noch 2 h bei 5°C rühren, gibt dann 300 ml Methylenchlorid zu und neutralisiert mit 76 g Kaliumcarbonat x 1.5 H₂O. Man rührt 18 h bei Raumtemperatur, saugt ab und engt das Filtrat bei 20°C im Vakuum ein.

Der Rückstand (11.2 g) wird in Ethanol gelöst. Durch Zugabe von 2.8 g Fumarsäure erhält man 5.9 g Fumarat der Titelverbindung vom Schmelzpunkt 123-127°C, d.s. 47% d.Th.

In analoger Weise werden erhalten:
1) 1-Methyl-N-(3-nitroxy-propyl)-piperidin-4-carbonsäureamid fumarat aus 1-Methyl-N-(3-hydroxy-propyl)-piperidin-4-carbonsäureamid oxalat
   Ausbeute: 31 %; Lösungsmittel: Ethanol; Schmp. 111-113°C
2) N-(1-Methyl-piperidin-4-yl)-4-nitroxy-buttersäureamid fumarat aus N-(1-Methyl-piperidin-4-yl)-4-hydroxy-buttersäureamid oxalat
   Ausbeute: 70 %; Lösungsmittel: Ethylacetat; Schmp. 120-125°C
3) trans-N-(1-Methyl-piperidin-4-yl)-4-nitroxy-cyclohexancarbonsäureamid aus trans-N-(1-Methyl-piperidin-4-yl)-4-hydroxy-cyclohexancarbonsäureamid oxalat
   Ausbeute: 65 %; Lösungsmittel: Dichlormethan; Schmp. 181-183°C
4) 4-Nitroxy-piperidin fumarat aus 4-Hydroxy-piperidin oxalat
   Ausbeute: 39 %; Lösungsmittel: Ethylacetat; Schmp. 180-185°C
5) 2-Amino-essigsäure-(4-nitroxy)piperidid fumarat aus 2-Amino-essigsäure-(4-hydroxy)-piperidid oxalat
   Ausbeute: 73 %; Lösungsmittel: Ethylacetat; Schmp. 112-115°C
6) 2-Dimethylamino-essigsäure-(4-nitroxy)-piperidid oxalat aus 2-Dimethylamino-essigsäure-(4-hydroxy)-piperidid oxalat
   Ausbeute: 61 %; Lösungsmittel: (2-Propanol); Schmp. 160-162°C
7) 2-Amino-2-methyl-propionsäure-(4-nitroxy)-piperidid fumarat aus 2-Amino-2-methyl-propionsäure-(4-hydroxy)-piperidid oxalat
   Ausbeute: 48 %; Lösungsmittel: Ethanol; Schmp. 185°C
8) 2-Amino-2-methyl-propionsäure-(4-nitroxymethyl)-piperidid fumarat aus 2-Amino-2-methyl-propionsäure-(4-hydroxymethyl)-piperidid oxalat
   Ausbeute: 50 %; Lösungsmittel: Ethanol; Schmp. 103-105°C
9) 2-Amino-2-methyl-propionsäure-[4-(2-nitroxyethyl)]-piperidid fumarat aus 2-Amino-2-methyl-propionsäure-[4-(2-hydroxyethyl)]-piperidid oxalat
   Ausbeute: 53 %; Lösungsmittel: Ethanol; Schmp. 155-157°C
10) 3-Amino-propionsäure-(4-nitroxy)-piperidid fumarat aus 3-Amino-propionsäure-(4-hydroxy)-piperidid oxalat
   Ausbeute: 64 %; Lösungsmittel: Ethanol; Schmp. 141-142°C
11) 3-Amino-propionsäure-(4-nitroxy-methyl)-piperidid fumarat aus 3-Amino-propionsäure-(4-hydroxy-methyl)-piperidid oxalat
   Ausbeute: 64 %; Lösungsmittel: Ethanol; Schmp. 141-142°C
12) 3-Dimethylamino-propionsäure-(4-nitroxy)-piperidid oxalat aus 3-Dimethylamino-propionsäure-(4-hydroxy)-piperidid oxalat
   Ausbeute: 29 %; Lösungsmittel: (2-Propanol); Schmp. 130-132°C
13) 4-Amino-4-methyl-valeriansäure-(4-nitroxy)-piperidid fumarat aus 4-Amino-4-methyl-valeriansäure-(4-hydroxy)-piperidid oxalat
   Ausbeute: 53 %; Lösungsmittel: Ethylacetat; Schmp. 263-265°C
14) 4-Amino-4-methyl-valeriansäure-(4-nitroxymethyl)-piperidid fumarat aus 4-Amino-4-methyl-valeriansäure-(4-hydroxymethyl)-piperidid oxalat
   Ausbeute: 28 %; Lösungsmittel: Ethylacetat/Aceton; Schmp. 142-145°C
15) 1-(2-Amino-ethyl)-4-nitroxy-piperidin fumarat aus 1-(2-Amino-ethyl)-4-hydroxy-piperidin oxalat
   Ausbeute: 60 %; Lösungsmittel: Ethanol; Schmp. 167°C
16) 1-(2-Amino-ethyl)-4-nitroxymethyl-piperidin difumarat aus 1-(2-Amino-ethyl)-4-hydroxymethyl-piperidin oxalat
   Ausbeute: 35 %; Lösungsmittel: Ethanol; Schmp. 150-153°C
17) 1-(3-Amino-propyl)-4-nitroxy-piperidin fumarat aus 1-(3-Amino-propyl)-4-hydroxy-piperidin oxalat
   Ausbeute: 35 %; Lösungsmittel: Ethanol; Schmp. 150-154°C
18) 1-(3-Amino-propyl)-4-nitroxymethyl-piperidin fumarat aus 1-(3-Amino-propyl)-4-hydroxymethyl-piperidin oxalat
   Ausbeute: 40 %; Lösungsmittel: Ethanol; Schmp. 128-130°C
19) 2,2-Dimethyl-3-nitroxy-N-(1-methyl-piperidin-4-yl)-propionsäureamid fumarat aus 2,2-Dimethyl-3-hydroxy-N-(1-methyl-piperidin-4-yl)-propionsäureamid oxalat
   Ausbeute: 20 %; Lösungsmittel: Ethanol; Schmp. 180-183°C
20) 5-Nitroxy-N-(1-methyl-piperidin-4-yl)-valeriansäureamid fumarat aus 5-Hydroxy-N-(1-methyl-piperidin-4-yl)-valeriansäureamid oxalat
   Ausbeute: 15 %; Lösungsmittel: Ethanol; Schmp. 102-106°C
21) 1-(2-Amino-ethyl)-4-(2-nitroxy-ethyl)-piperidin difumarat aus 1-(2-Amino-ethyl)-4-(2-hydroxy- ethyl)-piperidin oxalat
   Ausbeute: 20 %; Lösungsmittel: Ethanol; Schmp. 140-142°C
22) (S)-N-(2-Nitroxy-ethyl)-pyrrolidin-2-carbonsäureamid fumarat aus (S)-N-(2-Hydroxy-ethyl)-pyrrolidin-2-carbonsäureamid oxalat
   Ausbeute: 25 %; Lösungsmittel: Ethanol; Schmp. 125-126°C
23) 1-Methyl-N-(2-nitroxy-ethyl)-piperidin-2-carbonsäureamid fumarat aus 1-Methyl-N-(2-hydroxy-ethyl)-piperidin-2-carbonsäureamid
   Ausbeute: 45 %; Lösungsmittel: Ethanol; Schmp. 120-122°C
24) 1-Methyl-N-(2-nitroxy-ethyl)-piperidin-3-carbonsäureamid fumarat aus 1-Methyl-N-(2-hydroxy-ethyl)-piperidin-3-carbonsäureamid oxalat
   Ausbeute: 56 %; Lösungsmittel: Ethanol; Schmp. 99-102°C
25) 3-Amino-propionsäure-[4-(2-nitroxy-ethyl)]-piperidid fumarat aus 3-Amino-propionsäure-[4-(2-hydroxy-ethyl)]-piperidid oxalat
   Ausbeute: 11 %; Lösungsmittel: Ethanol; Schmp. 128-130°C
26) 4-Amino-4-methyl-valerian-säure-[4-(2-nitroxyethyl)]-piperidid fumarat aus 4-Amino-4-methyl-valeriansäure-[4-(2-hydroxyethyl)]-piperidid
   Ausbeute: 70 %; Lösungsmittel: Ethanol; Schmp. 150-152°C
27) Piperidin-4-carbonsäure-(4-nitroxy)-piperidid fumarat aus Piperidin-4-carbonsäure-(4-hydroxy)-piperidid
   Ausbeute: 60 %; Lösungsmittel: Ethanol; Schmp. 164-168°C
28) 4-(2-Nitroxyethyl)-piperidin cyclaminat aus 4-(2-Hydroxyethyl)-piperidin oxalat
   Ausbeute: 60 %; Lösungsmittel: Ethylacetat; Schmp. 82-83°C
29) 2-(2-Nitroxyethyl)-piperidin cyclaminat aus 2-(2-Hydroxyethyl)-piperidin oxalat
   Ausbeute: 90 %; Lösungsmittel: Ethylacetat;
   Schmp. 131-133°C
30) 4-Nitroxybuttersäure-N-(piperidin-4-yl)-amid cyclaminat aus 4-Hydroxybuttersäure-N-(piperidin-4-yl)-amid
   Ausbeute: 15 %; Lösungsmittel: Ethylacetat/Ethanol;
   Schmp. 113-115°C
31) 2-Dimethylamino-essigsäure-(4-nitroxy-methyl)-piperidid fumarat aus 2-Dimethylamino-essigsäure-(4-hydroxy-methyl)-piperidid oxalat
   Ausbeute: 73 %; Lösungsmittel: Ethanol; Schmp. 133-135°C
32) trans-4-Nitroxy-cyclohexan-carbonsäure-N-(piperidin-4-yl)-amid cyclaminat aus trans-4-Hydroxy-cyclohexan-carbon-säure N-(piperidin-4-yl)-amid
   Ausbeute: 27 %; Lösungsmittel: Diethylether; Schmp. 153-155°C
33) 1-(n-Butyl)-N-(2-nitroxy-ethyl)-piperidin-4-carbonsäure-amid fumarat aus 1-(n-Butyl)-N-(2-hydroxy-ethyl)-piperidin-4-carbonsäureamid
   Ausbeute: 63 %; Lösungsmittel: Ethanol/Diethylether; Schmp. 109-110°C
34) 1-(2-Methyl-1-propyl)-N-(2-nitroxyethyl)-piperidin-4-carbonsäureamid fumarat aus 1-(2-Methyl-1-propyl)-N-(2-hydroxyethyl)-piperidin-4-carbonsäureamid
   Ausbeute: 55 %; Lösungsmittel: Ethanol; Schmp. 119-121°C
35) 1-Methyl-piperidin-4-carbonsäure-(4-nitroxy)-piperidid fumarat aus 1-Methyl-piperidin-4-carbonsäure-(4-hydroxy)-piperidid
   Ausbeute: 74 %; Lösungsmittel: Ethanol; Schmp. 173-175°C

### Beispiel 2

### 4-Nitroxymethyl-piperidin nitrat

5.8 g 4-Hydroxymethyl-piperidin werden in 100 ml Acetonitril gelöst und auf -25 °C abgekühlt. Durch Zugabe von 3.1 ml 100proz. Salpetersäure wird die Base in das salpetersaure Salz überführt.

Zu dieser Lösung gibt man eine frisch bereitete Acetylnitrat-Lösung (aus 9.5 ml Essigsäureanhydrid und 4.2 ml 100proz. Salpetersäure in 40 ml Acetonitril bei -25 °C) zu und läßt noch 3 h bei dieser Temperatur rühren.
Die Reaktionslösung wird mit 300 ml Diisopropylether verdünnt. Das Kristallisat wird abgesaugt und mit Diethylether gewaschen. Man erhält 9.5 g (82 % d. Th.) Nitrat der Titelverbindung vom Schmelzpunkt 64-65 °C.

### Beispiel 3

### 1-Isopropylaminocarbonyl-4-nitroxy-piperidin

7 g 4-Nitroxy-piperidin werden in 25 ml Ethylacetat gelöst und unter Kühlen mit Eiswasser bei 0-5 °C mit 5.2 ml Isopropylisocyanat versetzt. Man rührt 2 h bei 10 °C und engt im Vakuum ein. Der Rückstand wird in Ether aufgenommen, die ausgefallenen Kristalle werden abgesaugt. Man erhält 6.6 g der Titelverbindung vom Schmelzpunkt 93-95 °C, d.s. 60% d.Th.

### Beispiel 4

### 1-Aminocarbonyl-4-nitroxy-piperidin

7.1 g 4-Nitroxy-piperidin werden in 60 ml Wasser und 30 ml Methanol gelöst und mit 16.2 g Kaliumcyanat versetzt. Nach Zutropfen von 12 ml Eisessig läßt man noch 4 h bei Raumtemperatur rühren. Durch Zugabe von 100 ml Ethylacetat werden die anorganischen Salze ausgefällt. Man saugt ab, trennt von der wäßrigen Phase und trocknet die organische Phase mit Natriumsulfat. Nach dem Absaugen wird im Vakuum eingeengt. Der Rückstand wird mit Ether verrieben und die Kristalle abgesaugt. Es verbleiben 8.3 g der Titelverbindung vom Schmelzpunkt 110-111 °C, d.s. 89% d.Th.

In analoger Weise wurden erhalten:
1) 1-Aminocarbonyl-4-nitroxymethyl-piperidin aus 4-Nitroxymethyl-piperidin und Kaliumcyanat
   Ausbeute: 55 %; Lösungsmittel: Diethylether; Schmp. 95-97°C
2) 1-Aminocarbonyl-4-(2-nitroxy-ethyl)piperidin aus 4-(2-Nitroxy-ethyl)piperidin und Kaliumcyanat
   Ausbeute: 75 %; Lösungsmittel: Diethylether; Schmp. 74-76°C
3) N-(1-Aminocarbonyl-piperidin-4-yl)-4-nitroxy-buttersäureamid aus 4-Nitroxy-buttersäure-(piperidin-4-yl)-amid
   Ausbeute: 25 %; Lösungsmittel: Ethanol; Schmp. 106-107°C
4) N-(1-Aminocarbonyl-piperidin-4-yl)-2,2-dimethyl-3-nitroxypropionsäureamid aus 2,2-Dimethyl-3-nitroxy-propionsäure-(piperidin-4-yl)-amid
   Ausbeute: 83 %; Lösungsmittel: Ethylacetat; Schmp. 142-143°C
5) N-(1-Aminocarbonyl-piperidin-4-yl)-5-nitroxy-valeriansäureamid aus 5-Nitroxy-valeriansäure-(piperidin-4-yl)-amid
   Ausbeute: 49 %; Lösungsmittel: Ethylacetat/Diethylether; Schmp. 117-118°C
6) trans-N-(1-Aminocarbonyl-piperidin-4-yl)-4-nitroxy-cyclohexancarbonsäureamid aus trans-4-Nitroxy-cyclohexancarbonsäure-(piperidin-4-yl)-amid
   Ausbeute: 25 %; Lösungsmittel: Ethanol; Schmp. 187-188°C

### Beispiel 5

### 1-(2-Aminocarbonylamino-ethyl)-4-nitroxy-piperidin

1.53 g 1-(2-Amino-ethyl)-4-nitroxy-piperidin fumarat werden in 15 ml Methanol und 5 ml Wasser gelöst. Man gibt 1.62 g Kaliumcyanat zu und rührt 10 Minuten bei Raumtemperatur. Dann fügt man 0.6 ml Essigsäure zu und rührt über Nacht bei Raumtemperatur. Man engt im Vakuum ein, der Rückstand wird in Wasser gelöst. Durch Zugabe von Kaliumcarbonat stellt man einen pH-Wert von 10 ein. Man extrahiert mit Ethylacetat und trocknet mit Natriumsulfat. Nach dem Einengen und Verreiben des Rückstandes mit Ether saugt man die entstandenen Kristalle ab. Es verbleiben 1 g der Titelverbindung vom Schmelzpunkt 114-116 °C, d.s. 86% d.Th.

### Beispiel 6

### 1-Ethyl-4-nitroxy-piperidin fumarat

6.9 g 4-Nitroxy-piperidin werden in 50 ml Aceton gelöst, mit 13.8 g Kaliumcarbonat und 6.1 ml Ethyliodid versetzt und 18 h bei Raumtemperatur gerührt. Nach dem Absaugen wird das Filtrat eingeengt, der Rückstand in Ethylacetat gelöst und 2mal mit Natriumchlorid-Lösung extrahiert. Die organische Phase wird mit Natriumsulfat getrocknet. Das nach Absaugen und Einengen verbleibende Rohprodukt (8.2 g) wird nach Lösen in Ethanol durch Zugabe von 2.7 g Fumarsäure in das Salz überführt. Nach Absaugen verbleiben 2.6 g Fumarat der Titelverbindung vom Schmelzpunkt 131-134 °C, d.s. 23% d.Th.
1) 4-Nitroxy-1-propyl-piperidin fumarat aus 4-Nitroxy-piperidin und n-Propyljodid
   Ausbeute: 30 %; Lösungsmittel: Ethanol; Schmp. 122-125°C
2) 4-Nitroxy-buttersäure-N-(1-propyl-piperidin-4-yl)-amid fumarat aus 4-Nitroxy-buttersäure-N-(piperidin-4-yl)amid und n-Propyljodid
   Ausbeute: 10 %; Lösungsmittel: Ethylacetat; Schmp. 100-102°C
3) 4-Nitroxy-1-hexyl-piperidin fumarat aus 4-Nitroxy-piperidin und n-Hexylbromid
   Ausbeute: 50 %; Lösungsmittel: Ethanol; Schmp. 102-103°C

### Beispiel 7

### 1-(2-Hydroxy-propyl)-4-nitroxy-piperidin hemifumarat

6.9 g 4-Nitroxy-piperidin werden in 25 ml Ethanol gelöst, mit 10.5 ml 1,2-Epoxy-propan versetzt und 2 d bei Raumtemperatur gerührt. Der nach dem Einengen verbleibende Rückstand (11.4 g) wird in 10 ml Ethanol gelöst und mit 3.5 g Fumarsäure versetzt. Der Niederschlag wird abgesaugt und man erhält 6.3 g Hemifumarat der Titelverbindung vom Schmelzpunkt 135-138 °C, d.s. 50% d.Th.

### Beispiel 8

### 1-Methyl-4-(2-nitroxyethyl)-piperidin fumarat

Zu 100 ml einer 1 N NaH₂PO₃-Lösung, gibt man 3.4 g (0.02 mol) 4-(2-Nitroxyethyl)-piperidin, in 10 ml Dioxan zu. Unter Kühlen und Rühren werden nun bei +10°C, 10 ml einer 37proz. Formaldehyd-Lösung, in 10 Minuten zugetropft. Nach Rühren über Nacht bei Raumtemperatur wird mit K₂CO₃ alkalisch gestellt und mit Essigester extrahiert. Die organische Phase wird mit Na₂SO₄ getrocknet und im Vac. abdestilliert. Das verbleibende Öl wird in Ethanol gelöst und durch Zugabe von 1.2 g Fumarsäure in das Salz überführt. Nach Absaugen erhält man 2 g Fumarat der Titelverbindung vom Schmp. 105-107°C, d.S. 33 % d.Th..

### Beispiel 9

### a) 3-Amino-propionsäure-(4-hydroxy)-piperidid hemioxalat

113 g 2-Cyanessigsäureethylester werden in 1 l Methylenchlorid gelöst und mit 75.1 g 4-Hydroxy-piperidin versetzt. Nach 3 d rühren bei Raumtemperatur wird im Vacuum abdestilliert. Es verbleiben 153 g Rohprodukt von 2-Cyanessigsäure-(4-hydroxy)-piperidid, d. s. ca. 100 % d.Th..

61 g dieses Produkts werden in 300 ml Methanol gelöst, mit 300 ml flüssigem Ammoniak versetzt und mit 20 g Raney-Nickel bei 100 b Wasserstoffdruck und Raumtemperatur hydriert. Nach Absaugen des Katalysators wird das Lösungsmittel im Vacuum abdestilliert, der Rückstand in 400 ml Ethanol gelöst und mit 19.4 g Oxalsäure in 200 ml Ethanol versetzt. Nach Stehen über Nacht wird abgesaugt. Es verbleiben 64.2 g Hemioxalat der Titelverbindung vom Schmp. 170-172°C, d.s. 75 % d.Th..

### b) 4-Amino-4-methyl-valeriansäure-(4-hydroxy)-piperidid oxalat

Zu einer Lösung von 180 ml 2-Nitropropan in 25.8 ml 35proz. Benzyltrimethylammoniumhydroxid-Lösung und 100 ml Isobutanol tropft man bei ca. 35°C 181 ml Acrylsäuremethylester. Man läßt abkühlen und extrahiert mit Methylenchlorid und Wasser, trocknet die organische Phase mit Natriumsulfat und engt ein. Der Rückstand wird fraktioniert destilliert (Kp₃ = 84-87°C). Man erhalt 285 g (82 % d.Th.) 4-Methyl-4-nitro-valeriansäuremethylester.

175 g dieses Produkts werden mit 500 ml 2 N-Natronlauge versetzt und 4 h bei Raumtemperatur gerührt. Das Wasser wird abdestilliert und der feste Rückstand im Vacuumtrockenschrank getrocknet. Man erhält 183.1 g (100 % d.Th.) Natriumsalz von 4-Methyl-4-nitro-valeriansäure.

Zu einer Suspension dieses Salzes in 1 L Methylenchlorid gibt man unter Kühlung 208.2 g Phosphorpentachlorid. Man läßt 15 h bei Raumtemperatur rühren, saugt vom Ungelösten ab und engt das Filtrat ein. Es verbleiben 153 g (85 % d.Th.) eines Öls von 4-Methyl-4-nitro-valeriansäurechlorid.

Zu einer Lösung von 4-Hydroxy-piperidin in 340 ml Aceton, 35.7 g Natriumacetat und 218 ml Wasser tropft man bei 0-5°C eine Lösung von 39.1 g 4-Methyl-4-nitro-valeriansäurechlorid in 108 ml Aceton. Man läßt 15 h bei Raumtemperatur ruhren, destilliert das Lösungsmittel ab, nimmt den Rückstand mit 600 ml Methylenchlorid auf und wäscht zweimal mit je 30 ml gesättigter Natriumchlorid-Lösung. Die organische Phase wird getrocknet und eingeengt. Es verbleiben 57.9 g des Rohprodukts von 4-Methyl-4-nitro-valeriansäure-(4-hydroxy)-piperidid.

Obiges Rohprodukt wird in 600 ml Ethanol gelöst, im Autoklaven mit 600 ml Ammoniak und 8 g Raney-Nickel versetzt und 10 h bei 40°C und 80 b Wasserstoffdruck hydriert. Man entspannt, engt ein und verreibt den festen Rückstand mit Ethylacetat. Es verbleiben 33.8 g eines Feststoffes. Dieses wird in Isopropanol gelöst und in der Hitze mit 14.2 g Oxalsäure versetzt. Man läßt abkühlen und saugt ab. Man erhält 39.5 g (55 %) der Titelverbindung vom Schmelzpunkt 106-109°C.

### c) 5-Hydroxy-N-(1-methyl-piperidin-4-yl)-valeriansäureamid Oxalat

5,3 g (0,05 mol) 4-Amino-1-methylpiperidin werden mit 5,4 ml (0,06 mol) gamma Valerolacton 48 h auf 90°C erhitzt. Man löst die Mischung in 50 ml Wasser und schüttelt mit Methylenchlorid. Die wässrige Phase wird mit Kaliumcarbonat alkalisch gestellt und mit n-Butanol extrahiert. Die Rohbase wird in Ethanol gelöst, 3 g Oxalsäure zugegeben, von Unlöslichem abfiltriert und abdestilliert. Es verbleiben 8,5 Oxalat der Titelverbindung als viskoses halbfestes Produkt d.s. 56 % d.Th.

Analog werden aus ß-Butyrolacton erhalten:
c1) 4-Hydroxy-N-(1-methyl-piperidin-4-yl)-buttersäureamid Oxalat.
   35 % Ausbeute
   Fp 40° sintern (Ether)

### d) 1-Methyl-N-(2-hydroxy-ethyl)-piperidin-4-carbonsäureamid

17,1 g (0,1 mol) 1-Methyl-piperidin-4-carbonsäure-ethylester (Lit: J. Am. Chem. Soc. 74 (1952) 3831) werden mit 9 ml (0,15 mol) 2-Hydroxy-ethylamin 48 h auf 60°C erwärmt und über eine Kieselgelsäule mit Methylenchlorid/ Methanol (9:1) gereinigt. Es werden 9.5 g Base als viskoses Öl erhalten, d. s. 52 % d. Th..

Analog werden mit 3-Hydroxy-propylamin erhalten:
d1) 1-Methyl-N-(3-hydroxy-propyl)-piperidin-4-carbonsäureamid
   80 % Ausbeute, Fp 40°C.

### e) 2-Amino-2-methyl-propionsäure(4-hydroxy)-piperidid oxalat

3.8 g (18 mmol) 2-Azido-2-methyl-propionsäure-(4-hydroxy)-piperidid werden in 100 ml Methanol gelöst und über Raney-Nickel bei Raumtemperatur und 1 bar Wasserstoff, hydriert. Nach Absaugen und Eindampfen wird in Ethanol mit Oxalsäure das Salz hergestellt. Es werden 3.0 g Oxalat der Titelverbindung vom Schmp. 197°C erhalten (74 % d. Th.).

Analog werden erhalten
e1) 2-Amino-2-methyl-propionsäure(4-hydroxymethyl)-piperidid oxalat
   65 % Ausbeute; Schmp. 177 - 178°C (Ethanol).
e2) 2-Amino-2-methyl-propionsäure (4-hydroxyethyl)-piperidid oxalat
   63 % Ausbeute; Schmp. 179°C (Ethanol.

### f) 2-Azido-2-methyl-propionsäure-(4-hydroxymethyl)-piperidid

15 g (0,13 mol) 4-Hydroxymethyl-piperidin werden in 130 ml Wasser und 30 ml Dioxan gelöst und 19,4 g (0,13 mol) 2-Azido-2-methyl propionsäurechlorid (nach analog Lit. J. Am. Chem. Soc. 77 (1955) 112) zugetropft. Durch gleichzeitiges Zutropfen von 2 N Natronlauge wird der pH der Lösung auf 12 -12.5 gehalten. Nach Ende der Reaktion wird mit Ether extrahiert. Nach Trocknen mit Natriumsulfat und Einengen verbleiben 17,8 g Rohöl, d. S. 60 % d. Th. das ohne weitere Reinigung zur nächsten Stufe eingesetzt wird.

Analog werden mit 4-Hydroxyethylpiperidin erhalten:
f1) 2-Azido-2-methyl-propionsäure-(4-hydroxyethyl)-piperidid
   Öl, 56 % Ausbeute.
f2) mit 4- Hydroxypiperidin werden erhalten:
   2-Azido-2-methyl-propionsäure-(4-hydroxy)-piperidid
   Öl, 60 % Ausbeute.

*) à chaîne droite ou ramifiée.

## Patentansprüche

1. Verbindungen der Formel I 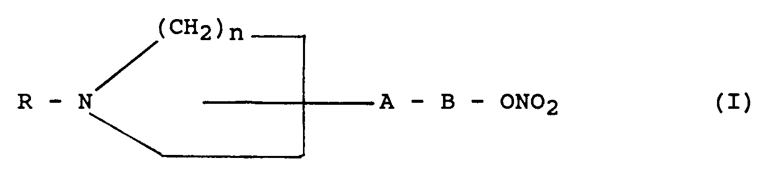 in der
n 1 oder 2 bedeutet,
R Wasserstoff, H-(C₁-C₈)-Alkylen, Hydroxy-(C₁-C₈)-Alkylen, R¹R²N-(C₁-C₈)-Alkylen, R¹R²N-(C₁-C₈)-Alkylen-CO-, R¹R²N-CO-NR³-(C₁-C₈)-Alkylen, oder R₁R₂N-CO- bedeutet, wobei (C₁-C₈)-Alkylen eine geradkettige, verzweigte oder cyclische Alkylenkette von 1 - 8 C-Atomen ist, worin
R¹ Wasserstoff oder eine geradkettige oder verzweigte, gesättigte oder ungesättigte, oder cyclische Alkyl-Gruppe von 1-6 C-Atomen bedeutet,
R² Wasserstoff oder eine geradkettige oder verzweigte, gesättigte oder ungesättigte, oder cyclische Alkyl-Gruppe von 1-6 C-Atomen bedeutet, oder R² gemeinsam mit einer -(CH₂)-Gruppe der benachbarten (C₁-C₈)-Alkyl-Gruppe und dem Stickstoff-Atom einen heteroaliphatischen Ring von 2-6 C-Atomen aufspannen kann,
R³ Wasserstoff oder eine geradkettige oder verzweigte, gesättigte oder ungesättigte, oder cyclische Alkyl-Gruppe von 1-6 C-Atomen bedeutet,
A eine Bindung, eine -NR⁴-CO-Gruppe oder eine -CO-NR⁴-Gruppe bedeutet, worin
R⁴ Wasserstoff oder eine geradkettige oder verzweigte, gesättigte oder ungesättigte oder cyclische Alkyl-Gruppe von 1-6 C-Atomen bedeutet,
B eine Bindung oder eine geradkettige oder verzweigte Alkylenkette von 1-8 C-Atomen, wobei eine -CH₂-Gruppe durch eine Cycloalkylen-Gruppe mit 3-7 C-Atomen ersetzt sein kann, bedeutet,
mit der Maßgabe, daß R keine R₁R₂N-(C₁-C₈)-Alkylen-CO-Gruppe sein kann, in der R₁ und R₂ gleichzeitig Wasserstoff bedeuten, und der Maßgabe, daß R keine H-(C₁-C₈)-Alkylgruppe bedeutet, wenn A und B gleichzeitig eine Bindung darstellen und n die Zahl 2 bedeutet,
sowie deren optisch aktive Formen und physiologisch verträgliche Salze.

2. Arzneimittel enthaltend mindestens eine Verbindung der Formel I 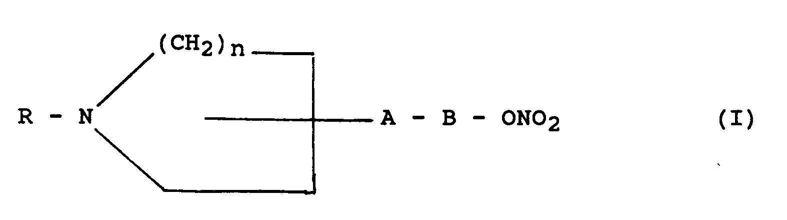 in der
n 1 oder 2 bedeutet,
R Wasserstoff, H-(C₁-C₈)-Alkylen, Hydroxy-(C₁-C₈)-Alkylen, R¹R²N-(C₁-C₈)-Alkylen, R¹R²N-(C₁-C₈)-Alkylen-CO-, R¹R²N-CO-NR³-(C₁-C₈)-Alkylen, oder R₁R₂N-CO- bedeutet, wobei (C₁-C₈)-Alkylen eine geradkettige, verzweigte oder cyclische Alkylenkette von 1-8 C-Atomen ist, worin
R¹ Wasserstoff oder eine geradkettige oder verzweigte, gesättigte oder ungesättigte, oder cyclische Alkyl-Gruppe von 1-6 C-Atomen bedeutet,
R² Wasserstoff oder eine geradkettige oder verzweigte, gesättigte oder ungesättigte, oder cyclische Alkyl-Gruppe von 1-6 C-Atomen bedeutet, oder R² gemeinsam mit einer -(CH₂)-Gruppe der benachbarten (C₁-C₈)-Alkyl-Gruppe und dem Stickstoff-Atom einen heteroaliphatischen Ring von 2-6 C-Atomen aufspannen kann,
R³ Wasserstoff oder eine geradkettige oder verzweigte, gesättigte oder ungesättigte, oder cyclische Alkyl-Gruppe von 1-6 C-Atomen bedeutet,
A eine Bindung, eine -NR⁴-CO-Gruppe oder eine -CO-NR⁴-Gruppe bedeutet, worin
R⁴ Wasserstoff oder eine geradkettige oder verzweigte, gesättigte oder ungesättigte oder cyclische Alkyl-Gruppe von 1-6 C-Atomen bedeutet,
B eine Bindung oder eine geradkettige oder verzweigte Alkylenkette von 1-8 C-Atomen, wobei eine -CH₂-Gruppe durch eine Cycloalkylen-Gruppe mit 3-7 C-Atomen ersetzt sein kann, bedeutet,
oder deren optisch aktive Formen oder deren physiologisch verträgliche Salze, sowie übliche pharmakologische Hilfs- und Trägerstoffe.

3. Arzneimittel gemäß Anspruch 2, wobei
R ein Wasserstoffatom; eine H-(C₁-C₃)-Alkylengruppe, beispielsweise Methyl, Ethyl, n-Propyl oder iso-Propyl; eine HO-(C₁-C₃)-Alkylengruppe, beispielsweise 2-Hydroxypropyl; eine R¹R²N-(C₁-C₃)-Alkylengruppe, beispielsweise Aminoethylen oder Aminopropylen; eine R¹R²N-(C₁-C₅)-Alkylencarbonyl-Gruppe, beispielsweise Aminomethylcarbonyl, Dimethylamino-methylcarbonyl, (2-Amino-1-methyl)-ethylcarbonyl, 2-Amino-ethylcarbonyl, 2-(N,N-Dimethylamino)-ethyl-carbonyl, (3-Amino-3-methyl)-butylcarbonyl, 1-Amino-ethyl-carbonyl oder (2-Amino-2-methyl)-propylcarbonyl; eine R¹R²N-CO-NR³-(C₁-C₃)-Alkylengruppe, beispielsweise Aminocarbonylaminoethyl; oder eine R¹R²N-CO-Gruppe, beispielsweise iso-Propylaminocarbonyl oder Aminocarbonyl bedeutet.

4. Arzneimittel gemäß Anspruch 2 oder 3, wobei
R₁ Wasserstoff oder eine geradkettige oder verzweigte C₁-C₃-Alkylgruppe, bespielsweise Methyl, Ethyl oder iso-Propyl, darstellt.

5. Arzneimittel gemäß einem der Ansprüche 2-4, wobei
R₂ Wasserstoff oder eine geradkettige C₁-C₃-Alkylgruppe, beispielsweise Methyl oder Ethyl bedeutet oder R₂ gemeinsam mit einer CH₂-Gruppe einer benachbarten Alkylengruppe und dem Stickstoffatom einen Piperidinring bildet.

6. Arzneimittel gemäß einem der Ansprüche 2-5, wobei
R₃ ein Wasserstoffatom darstellt.

7. Arzneimittel gemäß einem der Ansprüche 2-6, wobei
A eine Bindung, oder die Gruppe -NH-CO- oder -CO-NH- bedeutet.

8. Arzneimittel gemäß einem der Ansprüche 2-7, wobei
B eine Bindung, eine geradkettige oder verzweigte C₁-C₅-Alkylengruppe, wie beispielsweise Methylen, Ethylen, Trimethylen oder 1,1-Dimethyl-ethylen; oder eine 1,4-Cyclohexylengruppe darstellt.

9. Verfahren zur Herstellung von Verbindungen der Formel I 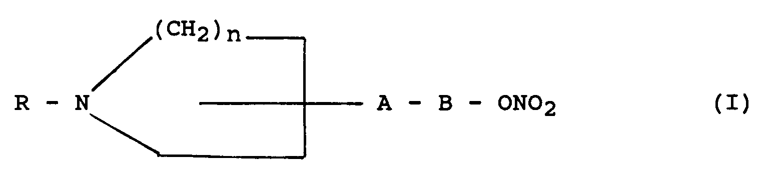 in der
n 1 oder 2 bedeutet,
R Wasserstoff, H-(C₁-C₈)-Alkylen, Hydroxy-(C₁-C₈)-Alkylen,R¹R²N-(C₁-C₈)-Alkylen, R¹R²N-(C₁-C₈)-Alkylen-CO-, R¹R²N-CO-NR³-(C₁-C₈)-Alkylen, oder R₁R₂N-CO- bedeutet, wobei (C₁-C₈)-Alkylen eine geradkettige, verzweigte oder cyclische Alkylenkette von 1-8 C-Atomen ist, worin
R¹ Wasserstoff oder eine geradkettige oder verzweigte, gesättigte oder ungesättigte, oder cyclische Alkyl-Gruppe von 1-6 C-Atomen bedeutet,
R² Wasserstoff oder eine geradkettige oder verzweigte, gesättigte oder ungesättigte, oder cyclische Alkyl-Gruppe von 1-6 C-Atomen bedeutet, oder R² gemeinsam mit einer -(CH₂)-Gruppe der benachbarten (C₁-C₈)-Alkyl-Gruppe und dem Stickstoff-Atom einen heteroaliphatischen Ring von 2-6 C-Atomen aufspannen kann,
R³ Wasserstoff oder eine geradkettige oder verzweigte, gesättigte oder ungesättigte, oder cyclische Alkyl-Gruppe von 1-6 C-Atomen bedeutet, A eine Bindung, eine -NR⁴-CO-Gruppe oder eine -CO-NR⁴-Gruppe bedeutet, worin
R⁴ Wasserstoff oder eine geradkettige oder verzweigte, gesättigte oder ungesättigte oder cyclische Alkyl-Gruppe von 1-6 C-Atomen bedeutet,
B eine Bindung oder eine geradkettige oder verzweigte Alkylenkette von 1-8 C-Atomen, wobei eine -CH₂-Gruppe durch eine Cycloalkylen-Gruppe mit 3-7 C-Atomen ersetzt sein kann, bedeutet,
mit der Maßgabe, daß R keine R₁R₂N-(C₁-C₈)-Alkylen-CO-Gruppe sein kann, in der R₁ und R₂ gleichzeitig Wasserstoff bedeuten, und der Maßgabe, daß R keine H-(C₁-C₈)-Alkylgruppe bedeutet, wenn A und B gleichzeitig eine Bindung darstellen und n die Zahl 2 bedeutet,
sowie deren optisch aktive Formen und physiologisch verträgliche Salze,
dadurch gekennzeichnet, daß man
a) eine Verbindung der allgemeinen Formel II 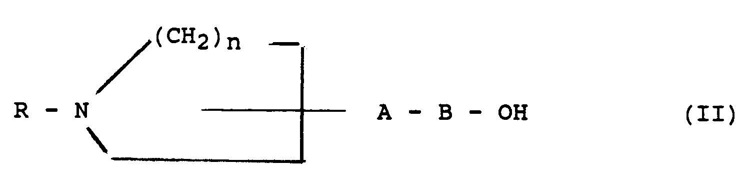 in der R, n, A und B die oben angegebenen Bedeutungen haben, einer Nitratester-Bildungsreaktion unterwirft, oder,
b) für den Fall, daß A eine -NR₄-CO-Gruppe darstellt, eine Verbindung der allgemeinen Formel III 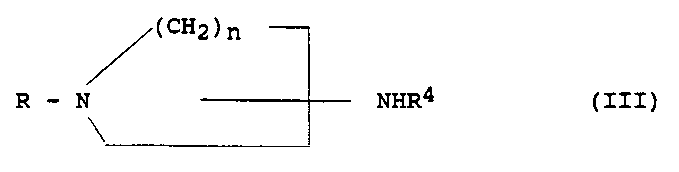 mit einer Verbindung der allgemeinen Formel IV
Z - CO - B - ONO₂ (IV)
wobei R, n, R⁴ und B die oben angegebenen Bedeutungen haben und Z eine nucleofuge Gruppe bedeutet, umsetzt, beziehungsweise
c) für den Fall, daß A eine -CO-NR⁴-Gruppe bedeutet, eine Verbindung der allgemeinen Formel V 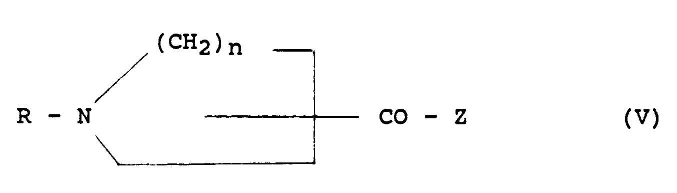 mit einer Verbindung der allgemeinen Formel VI
HNR⁴ - B - ONO₂ (VI)
wobei R, n, R⁴, B und Z die oben angebenen Bedeutungen haben, umsetzt, oder
d) im Fall, daß R nicht Wasserstoff bedeutet, eine Verbindung der allgemeinen Formel VII 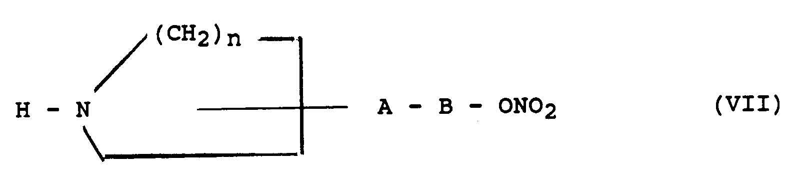 mit einer Verbindung der allgemeinen Formel VIII
R - Z (VIII)
wobei n, A, B, R und Z die oben an gegebenen Bedeutungen haben, umsetzt, oder
e) für den Fall, daß R die Gruppe R₁R₂N-CO- bedeutet, eine Verbindung der allgemeinen Formel VII mit Isocyanaten R₂-N=C=O oder Halogenameisensäure-Derivaten R²-NH-CO-Hal umsetzt, wobei R₂, A und B die oben angegebene Bedeutung haben und Hal, Chlor oder Brom bedeutet, oder
f) für den Fall, daß R die Gruppe HR²N-CO-NR³-(C₁-C₈)-Alkylen bedeutet, eine Verbindung der allgemeinen Formel IX 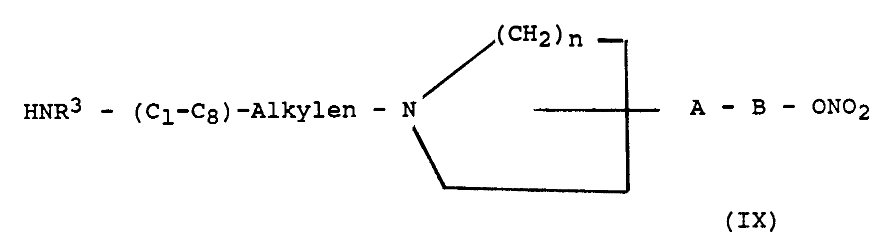 mit Isocyanaten R²-N=C=O oder Halogenameisensäure-Derivaten R²-NH-CO-Hal umsetzt, wobei R², R³, A, (C₁-C₈)-Alkylen und B die oben angegebenen Bedeutungen haben und Hal Chlor oder Brom bedeutet.

10. Arzneimittel enthaltend mindestens eine Verbindung gemäß Anspruch 1 sowie übliche pharmazeutische Träger- oder Hilfsstoffe.

11. Arzneimittel gemäß den Ansprüchen 2-8 oder 10 zur Behandlung von Herz- und Kreislauferkrankungen.

12. Verwendung von Verbindungen der Formel I gemäß den Ansprüchen 1-8 zur Herstellung von Arzneimitteln zur Behandlung von Herz- und Kreislauferkrankungen.

13. Verbindungen der Formel II 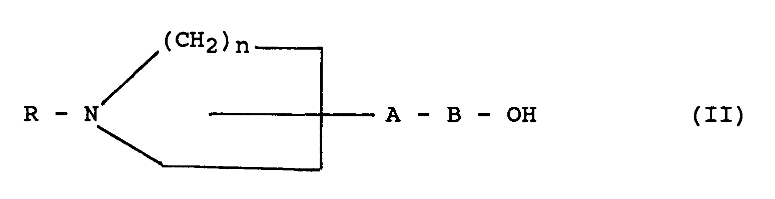 in der
n 1 oder 2 bedeutet,
R Wasserstoff, H-(C₁-C₈)-Alkylen, Hydroxy-(C₁-C₈)-Alkylen, R¹R²N-(C₁-C₈)-Alkylen, R¹R²N-(C₁-C₈)-Alkylen-CO-, R¹R²N-CO-NR³-(C₁-C₈)-Alkylen, oder R₁R₂N-CO- bedeutet, wobei (C₁-C₈)-Alkylen eine geradkettige, verzweigte oder cyclische Alkylenkette von 1-8 C-Atomen ist, worin
R¹ Wasserstoff oder eine geradkettige oder verzweigte, gesättigte oder ungesättigte, oder cyclische Alkyl-Gruppe von 1-6 C-Atomen bedeutet,
R² Wasserstoff oder eine geradkettige oder verzweigte, gesättigte oder ungesättigte, oder cyclische Alkyl-Gruppe von 1-6 C-Atomen bedeutet, oder R² gemeinsam mit einer -(CH₂)-Gruppe der benachbarten (C₁-C₈)-Alkyl-Gruppe und dem Stickstoff-Atom einen heteroaliphatischen Ring von 2-6 C-Atomen aufspannen kann,
R³ Wasserstoff oder eine geradkettige oder verzweigte, gesättigte oder ungesättigte, oder cyclische Alkyl-Gruppe von 1-6 C-Atomen bedeutet,
A eine Bindung, eine -NR⁴-CO-Gruppe oder eine -CO-NR⁴-Gruppe bedeutet, worin
R⁴ Wasserstoff oder eine geradkettige oder verzweigte, gesättigte oder ungesättigte oder cyclische Alkyl-Gruppe von 1-6 C-Atomen bedeutet,
B eine Bindung oder eine geradkettige oder verzweigte Alkylenkette von 1-8 C-Atomen, wobei eine -CH₂-Gruppe durch eine Cycloalkylen-Gruppe mit 3-7 C-Atomen ersetzt sein kann, bedeutet,
mit der Maßgabe, daß R keine R₁R₂N-(C₁-C₈) Alkylen-CO Gruppe sein kann, in der R₁ und R₂ gleichzeitig Wasserstoff bedeuten, der Maßgabe, daß R kein Wasserstoffatom oder eine H-(C₁-C₈)-Alkylengruppe bedeutet, wenn A eine Bindung darstellt und B eine Bindung oder eine Alkylenkette mit bis zu 2 C-Atomen bedeutet, und der Maßgabe, daß R keine CH₃- oder (CH₃)₃C-CH₂-Gruppe ist, wenn A eine Bindung, B eine Alkylenkette mit bis zu 2 C-Atomen und n gleich 1 bedeutet,
sowie deren optisch aktiven Formen.

14. Verwendung von Verbindungen der allgemeinen Formel II 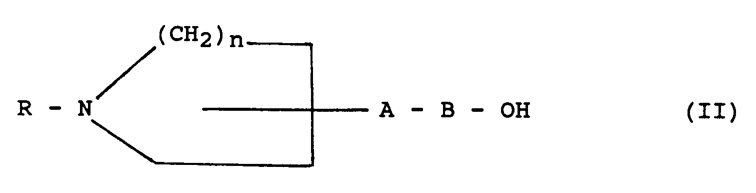 in der R, n, A und B die in den Ansprüchen 2-8 angegebenen Bedeutungen haben zur Herstellung von Verbindungen der Formel I gemäß den Ansprüchen 2-8.

15. Verfahren zur Herstellung eines Arzneimittels gemäß den Ansprüchen 2 bis 8, indem mindestens eine Verbindung der allgemeinen Formel I mit üblichen pharmazeutischen Träger- oder Hilfsstoffen formuliert wird.

## Claims

1. Compounds of the formula I 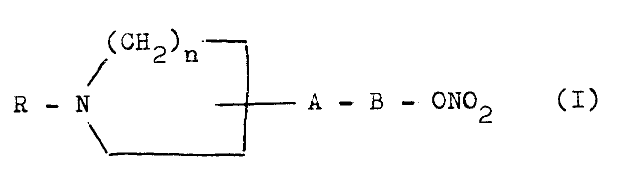 in which n signifies 1 or 2, R signifies hydrogen, H-(C₁-C₈)-alkylene, hydroxy-(C₁-C₈)-alkylene, R¹R²N-(C₁-C₈)-alkylene, R¹R²N-(C₁-C₈)-alkylene-CO-, R¹R²N-CO-NR³-(C₁-C₈)-alkylene or R¹R²N-CO-, whereby (C₁-C₈)-alkylene is a straight-chained or branched or cyclic alkylene chain of 1 - 8 C-atoms, wherein R¹ signifies hydrogen or a straight-chained or branched, saturated or unsaturated or cyclic alkyl group of 1 - 6 C-atoms, R² signifies hydrogen or a straight-chained or branched, saturated or unsaturated or cyclic alkyl group of 1 - 6 C-atoms or R², together with a -(CH₂)- group of the neighbouring (C₁-C₈)-alkyl group and the nitrogen atom, can bridge a heteroaliphatic ring of 2 - 6 C-atoms, R³ signifies hydrogen or a straight-chained or branched, saturated or unsaturated or cyclic alkyl group of 1 - 6 C-atoms, A signifies a bond, an -NR⁴-CO- group or a -CO-NR⁴ group, wherein R⁴ signifies hydrogen or a straight-chained or branched, saturated or unsaturated or cyclic alkyl group of 1 - 6 C-atoms, B signifies a bond or a straight-chained or branched alkylene chain of 1 - 8 C-atoms, whereby a -CH₂-group can be replaced by a cycloalkylene group with 3 - 7 C-atoms, with the proviso that R cannot be an R¹R²N-(C₁-C₈)-alkylene-CO- group in which R¹ and R² simultaneously signify hydrogen and with the proviso that R cannot signify a H-(C₁-C₈)-alkyl group when A and B simultaneously represent a bond and n signifies the number 2, as well as their optically-active forms and physiologically compatible salts.

2. Medicaments containing at least one compound of the formula I 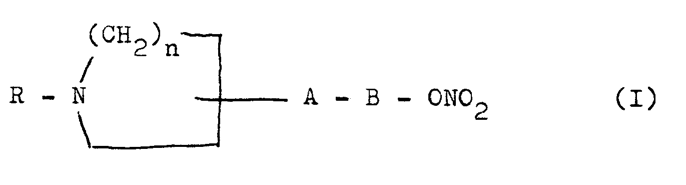 in which n signifies 1 or 2, R signifies hydrogen, H-(C₁-C₈)-alkylene, hydroxy-(C₁-C₈)-alkylene, R¹R²N-(C₁-C₈)-alkylene, R¹R²N-(C₁-C₈)-alkylene-CO, R¹R²N-CO-NR³-(C₁-C₈)-alkylene or R¹R²N-CO-, whereby (C₁-C₈)-alkylene is a straight-chained, branched or cyclic alkylene chain of 1 - 8 C-atoms, wherein R¹ signifies hydrogen or a straight-chained or branched, saturated or unsaturated or cyclic alkyl group of 1 - 6 C-atoms, R² signifies hydrogen or a straight-chained or branched, saturated or unsaturated or cyclic alkyl group of 1 - 6 C-atoms or R², together with a -(CH₂)- group of the neighbouring (C₁-C₈)-alkyl group and the nitrogen atom, can bridge a heteroaliphatic ring of 2 - 6 C-atoms, R³ signifies hydrogen or a straight-chained or branched, saturated or unsaturated or cyclic alkyl group of 1 - 6 C-atoms, A signifies a bond, an -NR⁴-CO- group or a -CO-NR⁴- group, wherein R⁴ signifies hydrogen or a straight-chained or branched- saturated or unsaturated or cyclic alkyl group of 1 - 6 C-atoms, B signifies a bond or a straight-chained or branched alkylene chain of 1 - 8 C-atoms, whereby a -CH₂- group can be replaced by a cycloalkylene group of 3 - 7 C-atoms, or their optically-active forms or their physiologically compatible salts, as well as usual pharmacological adjuvants and carrier materials.

3. Medicaments according to claim 2, whereby R signifies a hydrogen atom; an H-(C₁-C₃)-alkylene group, for example methyl, ethyl, n-propyl or isopropyl; an HO-(C₁-C₃)-alkylene group, for example 2-hydroxypropyl; an R¹R²N-(C₁-C₃)-alkylene group, for example aminoethylene or aminopropylene; an R¹R²N-(C₁-C₅)-alkylenecarbonyl group, for example aminomethylcarbonyl, dimethylaminomethylcarbonyl, (2-amino-1-methyl)-ethylcarbonyl, 2-aminoethylcarbonyl, 2-(N,N-dimethylamino)-ethylcarbonyl, (3-amino-3-methyl)-butylcarbonyl, 1-aminoethylcarbonyl or (2-amino-2-methyl)-propylcarbonyl; an R¹R²N-CO-NR³-(C₁-C₃)-alkylene group, for example aminocarbonylaminoethyl; or an R¹R²N-CO- group, for example isopropylaminocarbonyl or aminocarbonyl.

4. Medicaments according to claim 2 or 3, whereby R¹ represents hydrogen or a straight-chained or branched C₁-C₃-alkyl group, for example methyl, ethyl or isopropyl.

5. Medicaments according to one of claims 2 - 4, whereby R² signifies hydrogen or a straight-chained C₁-C₃-alkyl group, for example methyl or ethyl, or R², together with a -CH₂- group of a neighbouring alkylene group and the nitrogen atom, form a piperidine ring.

6. Medicaments according to one of claims 2 - 5, whereby R³ represents a hydrogen atom.

7. Medicaments according to one of claims 2 - 6, whereby A signifies a bond or the group -NH-CO- or -CO-NH-.

8. Medicaments according to one of claims 2 - 7, whereby B represents a bond, a straight-chained or branched C₁-C₅-alkylene group, such as for example methylene, ethylene, trimethylene or 1,1-dimethylethylene radical; or a 1,4-cyclohexylene group.

9. Process for the preparation of compounds of the formula I 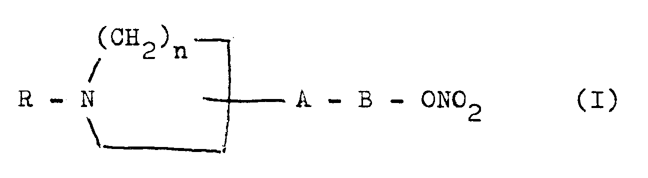 in which n signifies 1 or 2, R signifies hydrogen, H-(C₁-C₈)-alkylene, hydroxy-(C₁-C₈)-alkylene, R¹R²N-(C₁-C₈)-alkylene, R¹R²N-(C₁-C₈)-alkylene-CO-, R¹R²N-CO-NR³-(C₁-C₈)-alkylene or R¹R²N-CO-, whereby (C₁-C₈)-alkylene is a straight-chained, branched or cyclic alkylene chain of 1 - 8 C-atoms, R¹ signifies hydrogen or a straight-chained or branched, saturated or unsaturated or cyclic alkyl group of 1 - 6 C-atoms, R² signifies hydrogen or a straight-chained or branched, saturated or unsaturated or cyclic alkyl group of 1 - 6 C-atoms or R², together with a -(CH₂)- group of the neighbouring (C₁-C₈)-alkyl group and the nitrogen atom, can bridge a heteroaliphatic ring of 2 - 6 C-atoms, R³ signifies hydrogen or a straight-chained or branched, saturated or unsaturated or cyclic alkyl group of 1 - 6 C-atoms, A signifies a bond, an NR⁴-CO- group or a -CO-NR⁴ group, wherein R⁴ signifies hydrogen or a straight-chained or branched, saturated or unsaturated or cyclic alkyl group of 1 - 6 C-atoms, and B signifies a bond or a straight-chained or branched alkylene chain of 1 - 8 C-atoms, whereby a -CH₂- group can be replaced by a cycloalkylene group of 3 - 7 C-atoms, with the proviso that R cannot be an R¹R²N-(C₁-C₈)-alkylene-CO- group in which R¹ and R² simultaneously signify hydrogen and with the proviso that R does not signify an H-(C₁-C₈)-alkylene group when A and B simultaneously represent a bond and n signifies the number 2, as well as of their optically-active forms and physiologically compatible salts, characterised in that one
a) subjects a compound of the general formula II 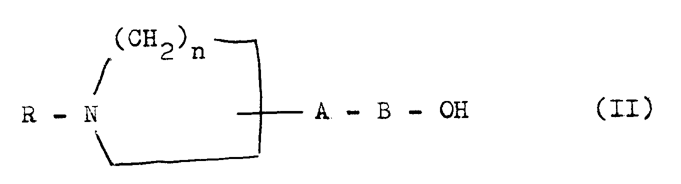 in which R, n, A and B have the above-given meanings, to a nitrate ester-formation reaction; or
b) for the case that A represents an -NR⁴-CO- group, reacts a compound of the general formula III 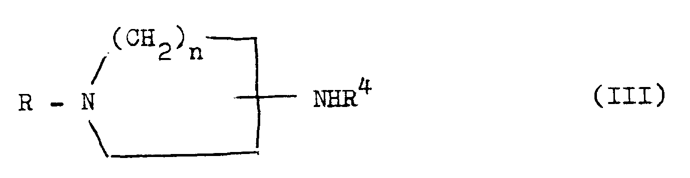 with a compound of the general formula IV
Z - CO - B - ONO₂ (IV)
whereby R, n, R⁴ and B have the above-given meaning and Z signifies a nucleofuge group, or
c) for the case that A signifies a -CO-NR⁴- group, reacts a compound of the general formula V 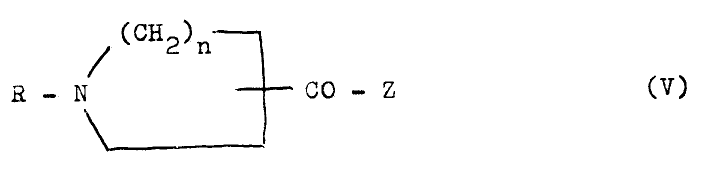 with a compound of the general formula VI
HNR⁴ - B - ONO₂ (VI)
whereby R, n, R⁴, B and Z have the above-given meanings, or
d) in that case that R does not represent hydrogen, reacts a compound of the general formula VII 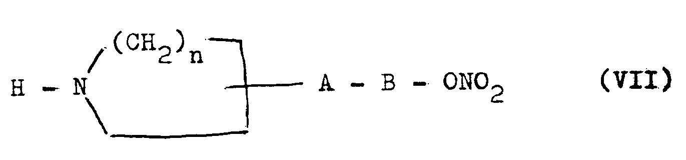 with a compound of the general formula VIII
R - Z (VIII)
whereby n, A, B, R and Z have the above-given meanings, or
e) for the case that R signifies the group R¹R²N-CO-, reacts a compound of the general formula VII with isocyanates R₂-N=C=O or haloformic acid derivatives R²-NH-CO-Hal, whereby R², A and B have the above-given meaning and Hal signifies chlorine or bromine, or
f) for the case that R signifies the group HR²N-CO-NR³-(C₁-C₈)-alkylene, reacts a compound of the general formula IX 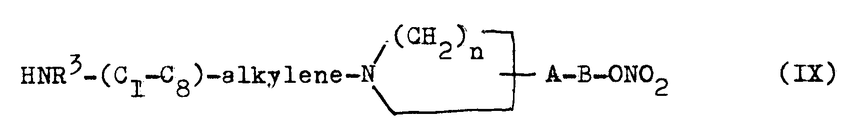 with isocynates R²-N=C=O or haloformic acid derivatives R²-NH-CO-Hal, whereby R², R³, A, (C₁-C₈)-alkylene and B have the above-given meanings and Hal signifies chlorine or bromine.

10. Medicaments containing at least one compound according to claim 1, as well as usual pharmaceutical carrier or adjuvant materials.

11. Medicaments according to claims 2 - 8 or 10 for the treatment of heart and circulatory diseases.

12. Use of compounds of the formula I according to claims 1 - 8 for the preparation of medicaments for the treatment of heart and circulatory diseases.

13. Compounds of the formula II 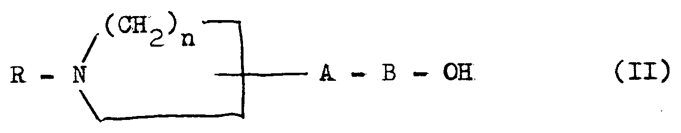 in which n signifies 1 or 2, R signifies hydrogen, H-(C₁-C₈)-alkylene, hydroxy-(C₁-C₈)-alkylene, R¹R²N-(C₁-C₈)-alkylene, R¹R²N-(C₁-C₈)-alkylene-CO-, R¹R²N-CO-NR³-(C₁-C₈)-alkylene or R¹R²N-CO-, whereby (C₁-C₈)-alkylene is a straight-chained or branched or cyclic alkylene chain of 1 - 8 C-atoms, wherein R¹ signifies hydrogen or a straight-chained or branched, saturated or unsaturated or cyclic alkyl group of 1 - 6 C-atoms, R² signifies hydrogen or a straight-chained or branched, saturated or unsaturated or cyclic alkyl group of 1 - 6 C-atoms or R², together with a -CH₂- group of the neighbouring (C₁-C₈)-alkyl group and the nitrogen atom, can bridge a heteroaliphatic ring of 2 - 6 C-atoms, R³ signifies hydrogen or a straight-chained or branched, saturated or unsaturated or cyclic alkyl group of 1 - 6 C-atoms, A signifies a bond, an -NR⁴-CO- group or a -CO-NR⁴- group, wherein R⁴ signifies hydrogen or a straight-chained or branched, saturated or unsaturated or cyclic alkyl group of 1 - 6 C-atoms, B signifies a bond or a straight-chained or branched alkylene chain of 1 - 8 C-atoms, whereby a -CH₂- group can be replaced by a cycloalkylene group of 3 - 7 C-atoms, with the proviso that R cannot be an R¹R²N-(C₁-C₈)-alkylene-CO- group in which R¹ and R² simultaneously signify hydrogen, with the proviso that R cannot be a hydrogen atom or an H-(C₁-C₈)-alkylene group when A represents a bond and B signifies a bond or an alkylene chain with up to 2 C-atoms and the proviso that R is not a CH₃- or (CH₃)₃C-CH₂- group when A signifies a bond, B an alkylene chain with up to 2 C-atoms and n is equal to 1, as well as their optically-active forms.

14. Use of compounds of the general formula II 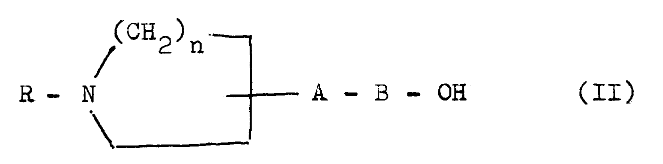 in which R, n, A and B have the meanings given in claims 2 - 8, for the preparation of compounds of the formula I according to claims 2 - 8.

15. Process for the preparation of medicaments according to claims claims 2 to 8, in which at least one compound of the general formula I is formulated with usual pharmaceutical carrier or adjuvant materials.

## Revendications

1. Composés de formule I 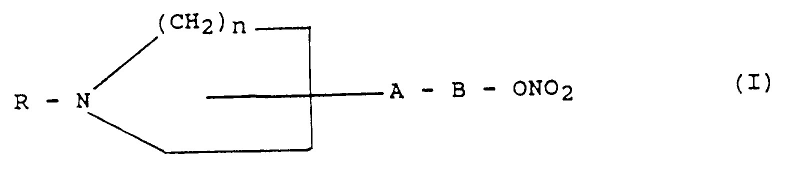 dans laquelle
n vaut 1 ou 2,
R représente un atome d'hydrogène, un groupe H-alkylène(C₁-C₈), hydroxy-alkylène(C₁-C₈), R¹R²N-alkylène(C₁-C₈), R¹R²N-alkylène(C₁-C₈)-CO-, R¹R²N-CO-NR³-alkylène(C₁-C₈) ou R₁R₂N-CO-, le groupe alkylène(C₁-C₈) représentant une chaîne alkylène droite, ramifiée ou cyclique, comportant 1-8 atomes de carbone, où
R¹ représente un atome d'hydrogène ou un groupe alkyle saturé ou insaturé, à chaîne droite ou ramifiée, ou cyclique, comportant 1-6 atomes de carbone,
R² représente un atome d'hydrogène ou un groupe alkyle saturé ou insaturé, à chaîne droite ou ramifiée, ou cyclique, comportant 1-6 atomes de carbone, ou R² peut constituer, conjointement avec un groupe -(CH₂)- du groupe alkyle(C₁-C₈) contigu et avec l'atome d'azote, un hétérocycle à 2-6 atomes de C,
R³ représente un atome d'hydrogène ou un groupe alkyle saturé ou insaturé, à chaîne droite ou ramifiée, ou cyclique, comportant 1-6 atomes de carbone,
A représente une liaison, un groupe -NR⁴-CO- ou un groupe -CO-NR⁴-, où
R⁴ représente un atome d'hydrogène ou un groupe alkyle saturé ou insaturé, à chaîne droite ou ramifiée, ou cyclique, comportant 1-6 atomes de carbone,
B représente une liaison ou un groupe alkylène à chaîne droite ou ramifiée, comportant 1-8 atomes de carbone, dans lequel un groupe -CH₂- peut être remplacé par un groupe cycloalkylène comportant 3-7 atomes de C,
étant entendu que R ne peut pas être un groupe R₁R₂N-alkylène(C₁-C₈)-CO- dans lequel R₁ et R₂ représentent simultanément l'hydrogène, et que R ne peut pas représenter un groupe H-alkyle(C₁-C₈), dans lequel A et B représentent simultanément une liaison et n vaut 2,
ainsi que leurs formes optiquement actives et leurs sels physiologiquement acceptables.

2. Médicament contenant au moins un composé de formule I 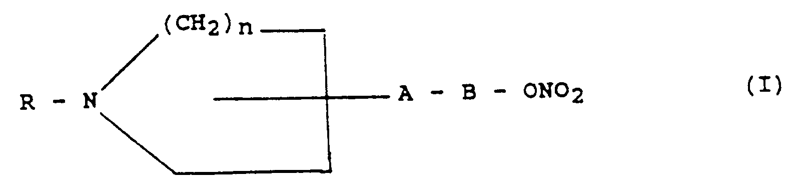 dans laquelle
n vaut 1 ou 2,
R représente un atome d'hydrogène, un groupe H-alkylène(C₁-C₈), hydroxy-alkylène(C₁-C₈), R¹R²N-alkylène(C₁-C₈), R¹R²N-alkylène(C₁-C₈)-CO-, R¹R²N-CO-NR³-alkylène(C₁-C₈) ou R₁R₂N-CO-, le groupe alkylène(C₁-C₈) représentant une chaîne alkylène droite, ramifiée ou cyclique, comportant 1-8 atomes de carbone, où
R¹ représente un atome d'hydrogène ou un groupe alkyle saturé ou insaturé, à chaîne droite ou ramifiée, ou cyclique, comportant 1-6 atomes de carbone,
R² représente un atome d'hydrogène ou un groupe alkyle saturé ou insaturé, à chaîne droite ou ramifiée, ou cyclique, comportant 1-6 atomes de carbone, ou R² peut former, conjointement avec un groupe -(CH₂)- du groupe alkyle(C₁-C₈) contigu et avec l'atome d'azote, un hétérocycle à 2-6 atomes de C,
R³ représente un atome d'hydrogène ou un groupe alkyle saturé ou insaturé, à chaîne droite ou ramifiée, ou cyclique, comportant 1-6 atomes de carbone,
A représente une liaison, un groupe -NR⁴-CO- ou un groupe -CO-NR⁴-, où
R⁴ représente un atome d'hydrogène ou un groupe alkyle saturé ou insaturé, à chaîne droite ou ramifiée, ou cyclique, comportant 1-6 atomes de carbone,
B représente une liaison ou un groupe alkylène à chaîne droite ou ramifiée, comportant 1-8 atomes de carbone, dans lequel un groupe -CH₂- peut être remplacé par un groupe cycloalkylène comportant 3-7 atomes de C,
ou ses formes optiquement actives ou ses sels physiologiquement acceptables, ainsi que des adjuvants et véhicules pharmacologiquement acceptables.

3. Médicament selon la revendication 2, dans lequel
R représente un atome d'hydrogène ; un groupe H-alkylène(C₁-C₃), par exemple un groupe méthyle, éthyle, n-propyle ou iso-propyle; un groupe HO-alkylène(C₁-C₃), par exemple un groupe 2-hydroxypropyle ; un groupe R¹R²N-alkylène(C₁-C₃), par exemple un groupe aminoéthylène ou aminopropylène ; un groupe R¹R²N-alkylène(C₁-C₅)-carbonyle, par exemple un groupe aminométhylcarbonyle, diméthylaminométhyl-carbonyle, (2-amino-1-méthyl)-éthylcarbonyle, 2-amino-éthylcarbonyle, 2-(N,N-diméthylamino)-éthyl-carbonyle, (3-amino-3-méthyl)-butylcarbonyle, 1-aminoéthylcarbonyle ou (2-amino-2-méthyl)-propylcarbonyle ; un groupe R¹R²N-CO-NR³-alkylène(C₁-C₃), par exemple aminocarbonylaminoéthyle ; ou un groupe R¹R²N-CO-, par exemple un groupe isopropylaminocarbonyle ou aminocarbonyle.

4. Médicament selon la revendication 2 ou 3, dans lequel
R₁ représente un atome d'hydrogène ou un groupe alkyle(C₁-C₃), *) à chaîne droite ou ramifiée, par exemple un groupe méthyle, éthyle ou iso-propyle.

5. Médicament selon l'une quelconque des revendications 1-4, dans lequel
R₂ représente un atome d'hydrogène ou un groupe alkyle(C₁-C₃) à chaîne droite, par exemple un groupe méthyle ou éthyle, ou R₂ forme, conjointement avec un groupe -CH₂- d'un groupe alkylène contigu et un atome d'azote, un cycle pipéridine.

6. Médicament selon l'une quelconque des revendications 2-5, dans lequel
R₃ représente un atome d'hydrogène.

7. Médicament selon l'une quelconque des revendications 2-6, dans lequel
A représente une liaison, ou un groupe -NH-CO- ou -CO-NH-.

8. Médicament selon l'une quelconque des revendications 2-7, dans lequel
B représente une liaison, un groupe alkylène(C₁-C₅) à chaîne droite ou ramifiée, par exemple un groupe méthylène, éthylène, triméthylène ou 1,1-diméthyl-éthylène ; ou un groupe 1,4-cyclohexylène.

9. Procédé de préparation de composés de formule I 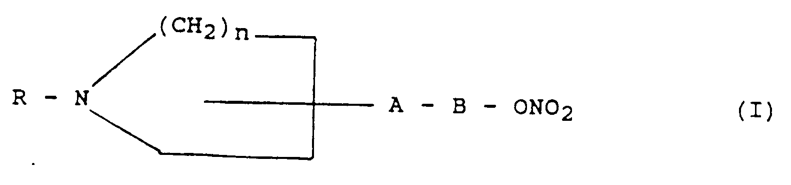 dans laquelle
n vaut 1 ou 2,
R représente un atome d'hydrogène, un groupe H-alkylène(C₁-C₈), hydroxy-alkylène(C₁-C₈), R¹R²N-alkylène(C₁-C₈), R¹R²N-alkylène(C₁-C₈)-CO-, R¹R²N-CO-NR³-alkylène(C₁-C₈) ou R₁R₂N-CO-, le groupe alkylène(C₁-C₈) représentant une chaîne alkylène droite, ramifiée ou cyclique, comportant 1-8 atomes de carbone, où
R¹ représente un atome d'hydrogène ou un groupe alkyle saturé ou insaturé, à chaîne droite ou ramifiée, ou cyclique, comportant 1-6 atomes de carbone,
R² représente un atome d'hydrogène ou un groupe alkyle saturé ou insaturé, à chaîne droite ou ramifiée, ou cyclique, comportant 1-6 atomes de carbone, ou R² peut constituer, conjointement avec un groupe -(CH₂)- du groupe alkyle(C₁-C₈) contigu et avec l'atome d'azote, un hétérocycle à 2-6 atomes de C,
R³ représente un atome d'hydrogène ou un groupe alkyle saturé ou insaturé, à chaîne droite ou ramifiée, ou cyclique, comportant 1-6 atomes de carbone,
A représente une liaison, un groupe -NR⁴-CO- ou un groupe -CO-NR⁴-, où
R⁴ représente un atome d'hydrogène ou un groupe alkyle saturé ou insaturé, à chaîne droite ou ramifiée, ou cyclique, comportant 1-6 atomes de carbone,
B représente une liaison ou un groupe alkylène à chaîne droite ou ramifiée, comportant 1-8 atomes de carbone, dans lequel un groupe -CH₂- peut être remplacé par un groupe cycloalkylène comportant 3-7 atomes de C,
étant entendu que R ne peut pas être un groupe R₁R₂N-alkylène(C₁-C₈)-CO- dans lequel R₁ et R₂ représentent simultanément l'hydrogène, et que R ne peut pas représenter un groupe H-alkyle(C₁-C₈), dans lequel A et B représentent simultanément une liaison et n vaut 2,
ainsi que leurs formes optiquement actives et leurs sels physiologiquement acceptables,
caractérisé en ce que
a) on soumet un composé de formule générale II 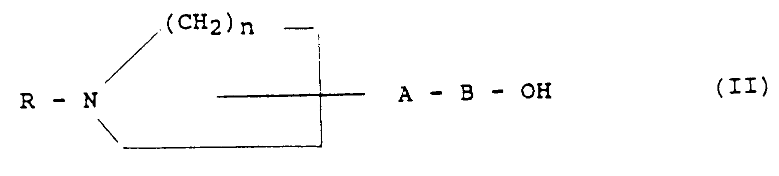 dans laquelle R, n, A et B ont les significations mentionnées ci-dessus, à une réaction de formation de nitrate-ester, ou
b) dans le cas où A représente un groupe -NR⁴-CO-, on fait réagir un composé de formule général III 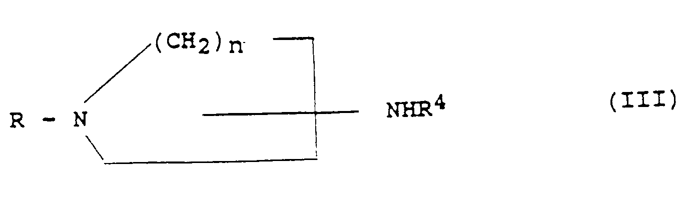 avec un composé de formule générale IV
Z-CO-B-ONO₂ (IV)
dans laquelle R, n, R⁴ et B ont les significations mentionnées plus haut, et Z représente un groupe nucléofuge, ou bien
c) dans le cas où A représente un groupe -CO-NR⁴-, on fait réagir un composé de formule générale V 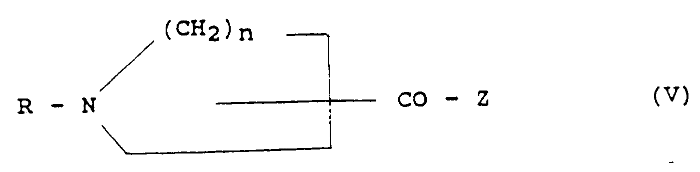 avec un composé de formule générale VI
HNR⁴-B-ONO₂ (VI)
dans laquelle R, n, R⁴, B et Z ont les significations mentionnées plus haut, ou
d) dans le cas où R ne représente pas l'hydrogène, on fait réagir un composé de formule générale VII 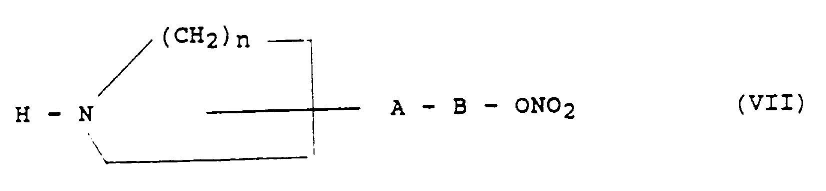 avec un composé de formule générale VIII
R-Z (VIII)
dans laquelle n, A, B, R et Z ont les significations mentionnées plus haut, ou
e) dans le cas où R représente le groupe R₁R₂N-CO-, on fait réagir un composé de formule générale VII avec des isocyanates R₂-N=C=O ou avec des dérivés d'acide halogénoformique R₂-NH-CO-Hal, R₂, A et B ayant les mêmes significations que plus haut, et Hal représentant le chlore ou le brome, ou
f) dans le cas où R représente un groupe HR²N-CO-NR³-alkylène(C₁-C₈), on fait réagir un composé de formule générale IX 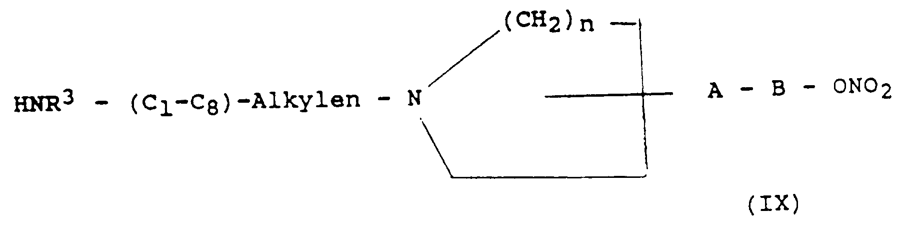 avec des isocyanates R²-N=C=O ou avec des dérivés d'acide halogénoformique R²-NH-CO-Hal, R², R³, A, alkylène(C₁-C₈) et B ayant les mêmes significations que plus haut, et Hal représentant le chlore ou le brome.

10. Médicament contenant au moins un composé selon la revendication 1, ainsi que des adjuvants ou véhicules pharmaceutiques usuels.

11. Médicament selon les revendications 2-8 ou 10, destiné au traitement de maladies cardiovasculaires.

12. Utilisation de composés de formule I selon les revendications 1-8, pour la préparation de médicaments destinés au traitement de maladies cardiovasculaires.

13. Composés de formule II 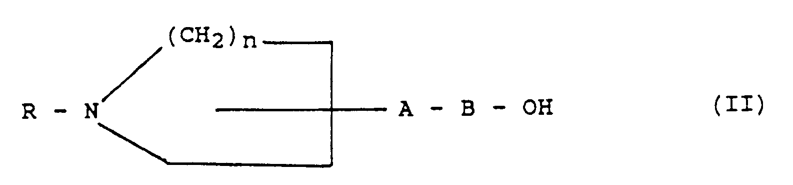 dans laquelle
n vaut 1 ou 2,
R représente un atome d'hydrogène, un groupe H-alkylène(C₁-C₈), hydroxy-alkylène(C₁-C₈), R¹R²N-alkylène(C₁-C₈), R¹R²N-alkylène(C₁-C₈)-CO-, R¹R²N-CO-NR³-alkylène(C₁-C₈) ou R₁R₂N-CO-, le groupe alkylène(C₁-C₈) représentant une chaîne alkylène droite, ramifiée ou cyclique, comportant 1-8 atomes de carbone, où
R¹ représente un atome d'hydrogène ou un groupe alkyle saturé ou insaturé, à chaîne droite ou ramifiée, ou cyclique, comportant 1-6 atomes de carbone,
R² représente un atome d'hydrogène ou un groupe alkyle saturé ou insaturé, à chaîne droite ou ramifiée, ou cyclique, comportant 1-6 atomes de carbone, ou R² peut constituer, conjointement avec un groupe -(CH₂)- du groupe alkyle(C₁-C₈) contigu et avec l'atome d'azote, un hétérocycle à 2-6 atomes de C,
R³ représente un atome d'hydrogène ou un groupe alkyle saturé ou insaturé, à chaîne droite ou ramifiée, ou cyclique, comportant 1-6 atomes de carbone,
A représente une liaison, un groupe -NR⁴-CO- ou un groupe -CO-NR⁴-, où
R⁴ représente un atome d'hydrogène ou un groupe alkyle saturé ou insaturé, à chaîne droite ou ramifiée, ou cyclique, comportant 1-6 atomes de carbone,
B représente une liaison ou un groupe alkylène à chaîne droite ou ramifiée, comportant 1-8 atomes de carbone, dans lequel un groupe -CH₂- peut être remplacé par un groupe cycloalkylène comportant 3-7 atomes de C,
étant entendu que R ne peut pas être un groupe R₁R₂N-alkylène(C₁-C₈)-CO- dans lequel R₁ et R₂ représentent simultanément l'hydrogène, et que R ne peut pas représenter un atome d'hydrogène ou un groupe H-alkylène(C₁-C₈), lorsque A représente une liaison et B représente une liaison ou une chaîne alkylène comportant jusqu'à 2 atomes de C, et que R ne peut pas représenter un groupe CH₃ ou (CH₃)₃C-CH₂, lorsque A représente une liaison, B une chaîne alkylène comportant jusqu'à 2 atomes de carbone et n vaut 1,
ainsi que leurs formes optiquement actives.

14. Utilisation de composés de formule générale II 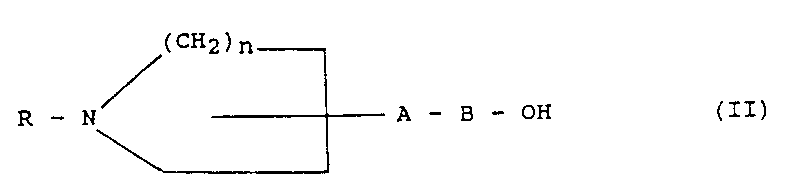 dans laquelle R, n, A et B ont les significations mentionnées dans les revendications 2-8, pour la préparation de composés de formule I selon les revendications 2-8.

15. Procédé de préparation d'un médicament selon les revendications 2 à 8, dans lequel au moins un composé de formule générale I est formulé avec des véhicules ou adjuvants pharmaceutiques usuels.
